# EUROPÄISCHE PATENTSCHRIFT

(11) **EP 2 313 495 B1**
(45) Veröffentlichungstag und Bekanntmachung des Hinweises auf die Patenterteilung: **02.12.2015**
(21) Anmeldenummer: 09772164.1
(22) Anmeldetag: 01.07.2009
(51) Int. Cl.: C12N 5/10, C12N 15/81, C12P 7/10, C12R 1/645, C12N 9/92, C12P 19/24

(54) **PROKARYOTISCHE XYLOSE-ISOMERASE ZUR KONSTRUKTION XYLOSE-VERGÄRENDER HEFEN**
PROKARYOTIC XYLOSE ISOMERASE FOR THE CONSTRUCTION OF XYLOSE FERMENTING YEASTS
XYLOSE-ISOMÉRASE PROCARYOTE POUR LA CONSTRUCTION DE LEVURES FERMENTANT LE XYLOSE

(30) Priorität: 02.07.2008 DE 102008031350
(43) Veröffentlichungstag der Anmeldung: 27.04.2011
(73) Patentinhaber: Lesaffre et Compagnie, 75001 Paris (FR)
(72) Erfinder: BRAT, Dawid, 65929 Frankfurt am Main (DE); BOLES, Eckhard, 64293 Darmstadt (DE); KELLER, Marco, 76879 Bornheim (DE); ROTHER, Beate, 37574 Einbeck (DE)
(74) Vertreter: Cabinet Plasseraud
(86) Internationale Anmeldenummer: PCT/EP2009/004762
(87) Internationale Veröffentlichungsnummer: WO 2010/000464

(56) Entgegenhaltungen:
- WO-A-03/062430
- WO-A-2007/089677
- WO-A-2008/141174
- US-B1- 6 475 768
- ANTONIUS J A VAN MARIS ET AL: "Alcoholic fermentation of carbon sources in biomass hydrolysates by Saccharomyces cerevisiae: current status" ANTONIE VAN LEEUWENHOEK, KLUWER ACADEMIC PUBLISHERS, DO, Bd. 90, Nr. 4, 11. Oktober 2006 (2006-10-11), Seiten 391-418, XP019446684 ISSN: 1572-9699
- KUYPER M ET AL: "High-level functional expression of a fungal xylose isomerase: The key to efficient ethanolic fermentation of xylose by Saccharomyces cerevisiae?" FEMS YEAST RESEARCH, ELSEVIER SCIENCE, TOKYO, NL, Bd. 4, Nr. 1, 1. Oktober 2003 (2003-10-01), Seiten 69-78, XP002312913 ISSN: 1567-1356
- KUYPER M ET AL: "Minimal metabolic engineering of Saccharomyces cerevisiae for efficient anaerobic xylose fermentation: a proof of principle" FEMS YEAST RESEARCH, ELSEVIER SCIENCE, TOKYO, NL, Bd. 4, Nr. 6, 1. März 2004 (2004-03-01), Seiten 655-664, XP002312911 ISSN: 1567-1356
- WALFRIDSSON M ET AL: "Ethanolic fermentation of xylose with Saccharomyces cerevisiae harboring the Thermus thermophilus xylA gene, which expresses and active xylose (glucose) isomerase" APPLIED AND ENVIRONMENTAL MICROBIOLOGY, AMERICAN SOCIETY FOR MICROBIOLOGY, US, Bd. 62, Nr. 12, 1. Dezember 1996 (1996-12-01), Seiten 4648-4651, XP002117639 ISSN: 0099-2240
- WIEDEMANN BEATE ET AL: "Codon-optimized bacterial genes improve L-Arabinose fermentation in recombinant Saccharomyces cerevisiae" APPLIED AND ENVIRONMENTAL MICROBIOLOGY, AMERICAN SOCIETY FOR MICROBIOLOGY, US, Bd. 74, Nr. 7, 1. April 2008 (2008-04-01), Seiten 2043-2050, XP002494170 ISSN: 1098-5336 [gefunden am 2008-02-08]
- JEFFRIES T W ET AL: "Metabolic engineering for improved fermentation of pentoses by yeasts" APPLIED MICROBIOLOGY AND BIOTECHNOLOGY, SPRINGER VERLAG, BERLIN, DE, Bd. 63, 1. Januar 2004 (2004-01-01), Seiten 495-509, XP002999147 ISSN: 0175-7598
- VAN MARIS A J A ET AL: "Development of Efficient Xylose Fermentation in Saccharomyces cerevisiae: Xylose Isomerase as a Key Component" ADVANCES IN BIOCHEMICAL ENGINEERING, BIOTECHNOLOGY, SPRINGER, BERLIN, DE, Bd. 108, 1. Januar 2007 (2007-01-01), Seiten 179-204, XP008086128 ISSN: 0724-6145
- WARNICK THOMAS A ET AL: "Clostridium phytofermentans sp. nov., a cellulolytic mesophile from forest soil" INTERNATIONAL JOURNAL OF SYSTEMATIC AND EVOLUTIONARY MICROBIOLOGY,, Bd. 52, Nr. 4, 1. Juli 2002 (2002-07-01), Seiten 1155-1160, XP002536553
- BRAT DAWID ET AL: "Functional Expression of a Bacterial Xylose Isomerase in Saccharomyces cerevisiae" APPLIED AND ENVIRONMENTAL MICROBIOLOGY, Bd. 75, Nr. 8, April 2009 (2009-04), Seiten 2304-2311, XP009121860 ISSN: 0099-2240

### SEQUENCE LISTING

<110> Johann Wolfgang Goethe-Universität Frankfurt am Main
<120> Prokaryotische Xylose-Isomerase zur Konstruktion Xylose-vergärender Hefen
<130> U30216PCT
<150> DE 10 2008 031 350.5
   <151> 2008-07-02
<160> 3
<170> PatentIn version 3.3
<210> 1
   <211> 438
   <212> PRT
   <213> Clostridium phytofermentans
<400> 1
   Met Lys Asn Tyr Phe Pro Asn Val Pro Glu Val Lys Tyr Glu Gly Pro
   1               5                   10                  15
   Asn Ser Thr Asn Pro Phe Ala Phe Lys Tyr Tyr Asp Ala Asn Lys Val
               20                  25                  30
   Val Ala Gly Lys Thr Met Lys Glu His Cys Arg Phe Ala Leu Ser Trp
           35                  40                  45
   Trp His Thr Leu Cys Ala Gly Gly Ala Asp Pro Phe Gly Val Thr Thr
       50                  55                  60
   Met Asp Arg Thr Tyr Gly Asn Ile Thr Asp Pro Met Glu Leu Ala Lys
   65                  70                  75                  80
   Ala Lys Val Asp Ala Gly Phe Glu Leu Met Thr Lys Leu Gly Ile Glu
                   85                  90                  95
   Phe Phe Cys Phe His Asp Ala Asp Ile Ala Pro Glu Gly Asp Thr Phe
               100                 105                 110
   Glu Glu Ser Lys Lys Asn Leu Phe Glu Ile Val Asp Tyr Ile Lys Glu
           115                 120                 125
   Lys Met Asp Gln Thr Gly Ile Lys Leu Leu Trp Gly Thr Ala Asn Asn
       130                 135                 140
   Phe Ser His Pro Arg Phe Met His Gly Ala Ser Thr Ser Cys Asn Ala
   145                 150                 155                 160
   Asp Val Phe Ala Tyr Ala Ala Ala Lys Ile Lys Asn Ala Leu Asp Ala
                   165                 170                 175
   Thr Ile Lys Leu Gly Gly Lys Gly Tyr Val Phe Trp Gly Gly Arg Glu
               180                 185                 190
   Gly Tyr Glu Thr Leu Leu Asn Thr Asp Leu Gly Leu Glu Leu Asp Asn
           195                 200                 205
   Met Ala Arg Leu Met Lys Met Ala Val Glu Tyr Gly Arg Ala Asn Gly
       210                 215                 220
   Phe Asp Gly Asp Phe Tyr Ile Glu Pro Lys Pro Lys Glu Pro Thr Lys
   225                 230                 235                 240
   His Gln Tyr Asp Phe Asp Thr Ala Thr Val Leu Ala Phe Leu Arg Lys
                   245                 250                 255
   Tyr Gly Leu Glu Lys Asp Phe Lys Met Asn Ile Glu Ala Asn His Ala
               260                 265                 270
   Thr Leu Ala Gly His Thr Phe Glu His Glu Leu Ala Met Ala Arg Val
           275                 280                 285
   Asn Gly Ala Phe Gly Ser Val Asp Ala Asn Gln Gly Asp Pro Asn Leu
       290                 295                 300
   Gly Trp Asp Thr Asp Gln Phe Pro Thr Asp Val His Ser Ala Thr Leu
   305                 310                 315                 320
   Ala Met Leu Glu Val Leu Lys Ala Gly Gly Phe Thr Asn Gly Gly Leu
                   325                 330                 335
   Asn Phe Asp Ala Lys Val Arg Arg Gly Ser Phe Glu Phe Asp Asp Ile
               340                 345                 350
   Ala Tyr Gly Tyr Ile Ala Gly Met Asp Thr Phe Ala Leu Gly Leu Ile
           355                 360                 365
   Lys Ala Ala Glu Ile Ile Asp Asp Gly Arg Ile Ala Lys Phe Val Asp
       370                 375                 380
   Asp Arg Tyr Ala Ser Tyr Lys Thr Gly Ile Gly Lys Ala Ile Val Asp
   385                 390                 395                 400
   Gly Thr Thr Ser Leu Glu Glu Leu Glu Gln Tyr Val Leu Thr His Ser
                   405                 410                 415
   Glu Pro Val Met Gln Ser Gly Arg Gln Glu Val Leu Glu Thr Ile Val
               420                 425                 430
   Asn Asn Ile Leu Phe Arg
           435
<210> 2
   <211> 1317
   <212> DNA
   <213> Clostridium phytofermentans
<400> 2
   atgaaaaatt actttccaaa tgttccagaa gtaaaatacg aaggcccaaa ttcaacgaat      60
   ccatttgctt ttaaatatta tgacgcaaat aaagttgtag cgggtaaaac aatgaaagag     120
   cactgtcgtt ttgcattatc ttggtggcat actctttgtg caggtggtgc tgatccattc     180
   ggtgtaacaa ctatggatag aacctacgga aatatcacag atccaatgga acttgctaag     240
   gcaaaagttg acgctggttt cgaattaatg actaaattag gaattgaatt cttctgtttc     300
   catgacgcag atattgctcc agaaggtgat acttttgaag agtcaaagaa gaatcttttt     360
   gaaatcgttg attacatcaa agagaagatg gatcagactg gtatcaagtt attatggggt     420
   actgctaata actttagtca tccaagattt atgcatggtg cttccacatc ttgcaacgca     480
   gacgtatttg catatgctgc tgctaagatt aagaatgcat tagatgcaac aattaaatta     540
   ggcggtaaag gttatgtatt ctggggtggt cgtgaaggtt atgaaacact tcttaataca     600
   gatttaggac ttgagcttga taatatggct agacttatga agatggctgt agagtatggc     660
   cgtgcaaatg gttttgatgg cgacttctat attgagccaa agccaaagga accaaccaag     720
   catcaatatg attttgatac agcaaccgta cttgctttcc ttcgcaaata tggcttagaa     780
   aaagatttca agatgaacat tgaagcaaac catgctactc ttgcaggtca tacctttgaa     840
   catgaacttg caatggctag agttaatggt gcatttggtt ctgtagatgc aaaccagggt     900
   gatccaaacc ttggatggga tacggatcaa ttcccaactg atgttcatag tgcaactctt     960
   gcaatgcttg aagtacttaa ggctggtgga ttcactaacg gcggacttaa ctttgatgca    1020
   aaggtaagac gtggttcctt cgaatttgat gatattgcat acggttatat tgcaggaatg    1080
   gatacttttg cacttggttt aattaaggct gctgagatta tcgacgatgg tagaatcgca    1140
   aaatttgtag atgatcgtta tgcaagctat aaaacaggaa ttggtaaagc aattgtggat    1200
   ggaactacat ctcttgaaga attagagcag tatgttttaa cacatagtga accagtaatg    1260
   cagagtggtc gtcaggaagt tcttgaaaca atcgtaaata atattttatt tagataa       1317
<210> 3
   <211> 1317
   <212> DNA
   <213> Artificial
<220>
   <223> Nukleinsäuresequenz des offenen Leserahmens (ORF) der Xylose-Isomerase aus C. phytofermentans in einer Codon-optimierten Form
<400> 3
   atgaagaact acttcccaaa cgttccagaa gttaagtacg aaggtccaaa ctctaccaac     60
   ccattcgctt tcaagtacta cgacgctaac aaggttgttg ctggtaagac catgaaggaa    120
   cactgtagat tcgctttgtc ttggtggcac accttgtgtg ctggtggtgc tgacccattc    180
   ggtgttacca ccatggacag aacctacggt aacattaccg acccaatgga attggctaag    240
   gctaaggttg acgctggttt cgaattgatg accaagttgg gtattgaatt cttctgtttc    300
   cacgacgctg acattgctcc agaaggtgac accttcgaag aatctaagaa gaacttgttc    360
   gaaattgttg actacattaa ggaaaagatg gaccaaaccg gtattaagtt gttgtggggt    420
   accgctaaca acttctctca cccaagattc atgcacggtg cttctacctc ttgtaacgct    480
   gacgttttcg cttacgctgc tgctaagatt aagaacgctt tggacgctac cattaagttg    540
   ggtggtaagg gttacgtttt ctggggtggt agagaaggtt acgaaacctt gttgaacacc    600
   gacttgggtt tggaattgga caacatggct agattgatga agatggctgt tgaatacggt    660
   agagctaacg gtttcgacgg tgacttctac attgaaccaa agccaaagga accaaccaag    720
   caccaatacg acttcgacac cgctaccgtt ttggctttct tgagaaagta cggtttggaa    780
   aaggacttca agatgaacat tgaagctaac cacgctacct tggctggtca caccttcgaa    840
   cacgaattgg ctatggctag agttaacggt gctttcggtt ctgttgacgc taaccaaggt    900
   gacccaaact tgggttggga caccgaccaa ttcccaaccg acgttcactc tgctaccttg    960
   gctatgttgg aagttttgaa ggctggtggt ttcaccaacg gtggtttgaa cttcgacgct   1020
   aaggttagaa gaggttcttt cgaattcgac gacattgctt acggttacat tgctggtatg   1080
   gacaccttcg ctttgggttt gattaaggct gctgaaatta ttgacgacgg tagaattgct   1140
   aagttcgttg acgacagata cgcttcttac aagaccggta ttggtaaggc tattgttgac   1200
   ggtaccacct ctttggaaga attggaacaa tacgttttga cccactctga accagttatg   1260
   caatctggta gacaagaagt tttggaaacc attgttaaca acattttgtt cagataa      1317

## Beschreibung

Die vorliegende Erfindung bezieht sich auf die Verwendung von Nukleinsäuremolekülen, die für eine bakterielle Xylose-Isomerase (XI) kodieren, die bevorzugt von *Clostridium phytofermentans* stammt, für die Umsetzung/Verstoffwechselung, dabei insbesondere Fermentation, mit rekombinanten Mikroorganismen von Xylose-haltigem Biomaterial und insbesondere die Herstellung von Bioalkoholen, dabei insbesondere Bioethanol, mittels Xylose-vergärender Hefen. Die vorliegende Erfindung bezieht sich weiterhin auf Zellen, insbesondere eukaryotische Zellen, die mit einem Nukleinsäure-Expressionskonstrukt, das eine Xylose-Isomerase (XI) kodiert, transformiert sind, wobei die Expression des Nukleinsäure-Expressionskonstrukts den Zellen die Fähigkeit verleiht, Xylose direkt zu Xylulose zu isomerisieren. Diese Zellen werden bevorzugt zur Umsetzung/Verstoffwechselung, dabei insbesondere Fermentation, von Xylose-haltigem Biomaterial und insbesondere zur Herstellung von Bioalkoholen, dabei insbesondere Bioethanol, verwendet. Die vorliegende Erfindung bezieht sich weiterhin auf Verfahren zur Produktion von Bioethanol und auf Verfahren zur Produktion von weiteren Verstoffwechselungsprodukten, umfassend die Verstoffwechselung von Medien, die Xylose enthalten.

### Hintergrund der Erfindung

Die Bier-, Wein- und Bäckerhefe *Saccharomyces cerevisiae* wird aufgrund ihrer Eigenschaft, Zucker zu Ethanol und Kohlendioxid zu fermentieren, schon seit Jahrhunderten für die Brot-, Wein- und Bierherstellung genutzt. In der Biotechnologie findet *S. cerevisiae* neben der Produktion von heterologen Proteinen vor allem in der Ethanolproduktion für industrielle Zwecke Verwendung. Ethanol wird in zahlreichen Industriezweigen als Ausgangssubstrat für Synthesen benutzt. Durch die immer knapper werdenden Ölvorkommen, die steigenden Ölpreise und den stetig steigenden weltweiten Benzinbedarf gewinnt Ethanol als Kraftstoffalternative mehr und mehr an Bedeutung.

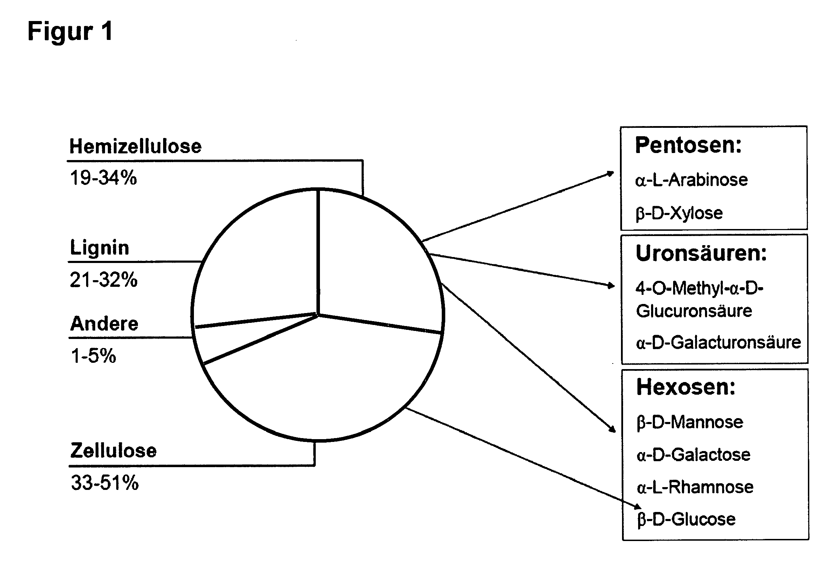

Um eine kostengünstige und effiziente Bioethanol-Produktion zu ermöglichen bietet sich die Verwendung von lignozellulosehaltiger Biomasse, wie z.B. Stroh, Abfälle aus der Holz- und Landwirtschaft und der organische Anteil aus dem alltäglichen Hausmüll, als Ausgangssubstrat an. Diese ist zum einen sehr günstig und zum anderen in großer Menge vorhanden. Die drei größten Bestandteile der Lignozellulose sind Lignin, Zellulose und Hemizellulose. Hemizellulose, nach Zellulose das am zweithäufigsten vorkommende Polymer, ist ein stark verzweigtes Heteropolymer. Es besteht aus Pentosen (L-Arabinose, D-Xylose), Uronsäuren (4-O-Methyl-D-Glucuronsäure, D-Galacturonsäure) und Hexosen (D-Mannose, D-Galactose, L-Rhamnose, D-Glucose) (siehe Figur 1). Hemizellulose lässt sich zwar leichter als Zellulose hydrolysieren, besitzt aber die Pentosen L-Arabinose und D-Xylose, die von der Hefe *S. cerevisiae* normalerweise nicht umgesetzt werden können.

Um Pentosen für Fermentationen nutzen zu können, müssen diese zuerst über die Plasmamembran in die Zelle hinein gelangen. Obwohl *S*. *cerevisiae* nicht in der Lage ist, D-Xylose zu metabolisieren, kann es diese in die Zelle aufnehmen. *S*. *cerevisiae* besitzt aber keine spezifischen Transporter. Der Transport findet mit Hilfe der zahlreichen Hexosetransporter statt. Die Affinität der Transporter zu D-Xylose ist aber deutlich niedriger als die zu D-Glucose (Kotter und Ciriacy, 1993). In Hefen, die D-Xylose metabolisieren können, wie z.B. *P. stipitis, C. shehatae* oder *P. tannophilus* (Du Preez et al., 1986), gibt es sowohl unspezifische niederaffine Transporter, die D-Glucose transportieren, als auch spezifische hochaffine Protonen-Symporter nur für D-Xylose (Hahn-Hagerdal *et al.,* 2001).

### Verwertung von D-Xylose

Verschiedene Bakterien, Hefen und Pilze sind in der Lage, Xylose zu verstoffwechseln. Die Verstoffwechselung von Xylose unterscheidet sich bei Pro- und Eukaryonten hauptsächlich in der Art der Isomerisierung der Xylose zu Xylulose. In Prokaryonten erfolgt die Umsetzung von Xylose zu Xylulose durch das Enzym Xylose-Isomerase (XI). In Eukaryonten wird Xylose meist in zwei Schritten isomerisiert. Zuerst wird Xylose durch die NAD(P)H-abhängige Xylose-Reduktase (XR) zu Xylitol reduziert und weiter durch die NAD-abhängige Xylitol-Dehydrogenase (XDH) zu Xylulose umgesetzt. Die anschließende Phosphorylierungsreaktion erfolgt in Pro- und Eukaryonten durch die Xylulokinase (XK).

Das entstandene Intermediat Xylulose-5-Phosphat ist ein Intermediat des Pentosephosphatweges. Der Hauptteil des Xylulose-5-Phosphats gelangt in Form von Fruktose-6-Phosphat und Glycerinaldehyd-3-Phosphat in die Glykolyse und wird dort weiter zu Pyruvat umgesetzt (Schaaff-Gerstenschläger und Miosga, 1997). Unter fermentativen Bedingungen wird der Zucker durch die Pyruvat-Decarboxylase und durch die Alkohol-Dehydrogenase weiter zu Ethanol abgebaut. Unter aeroben Bedingungen kann Pyruvat im Citratzyklus über eine Reihe von Reaktionsschritten bis zum Kohlendioxid oxidiert werden.

### Verwertung von D-Xylose in S. cerevisiae

In Arbeiten von Kötter und Ciriacy (1993) wurde erstmals ein rekombinanter *S*. *cerevisiae* Stamm konstruiert, der in der Lage war, D-Xylose zu metabolisieren. Hierfür wurden die für D-Xylose Reduktase (*XYL1*) und Xylitol Dehydrogenase (*XYL2*) kodierenden Gene der Hefe *Pichia stipitis* heterolog in der Hefe *S. cerevisiae* exprimiert. In späteren Arbeiten wurde zusätzlich die endogene Xylulokinase (*XKS1*) überexprimiert, was die D-Xylose Aufnahme in die Zelle sowie deren Umsetzung zu Ethanol verbesserte (Ho *et al.,* 1998; Eliasson *et al*., 2000). Trotz der erzielten Verbesserungen war unter Sauerstoff-limitierten Bedingungen das Hauptnebenprodukt der Xyloseumsetzung Xylitol. Dies wird einem Ungleichgewicht in der Redoxbalance zugeschrieben, welches dadurch verursacht wird, dass die in dem Stoffwechselweg zuerst ablaufende Reaktion bevorzugt NADPH nutzt, die zweite Reaktion jedoch ausschließlich NADH produziert (Hahn-Hägerdal *et al.,* 2001). Unter aeroben Bedingungen kann das durch die Xylitol-Dehydrogenase gebildete NADH über die Atmungskette zu NAD regeneriert werden. Unter anaeroben Bedingungen kann NAD nicht regeneriert werden und es kommt zu einer Ansammlung von NADH in der Zelle. Ohne den Cofaktor NAD kann die Xylitol-Dehydrogenase Xylitol nicht weiter zu Xylulose umsetzen.

Obwohl die in der genannten Arbeit verwendete Xylose-Reduktase aus *P. stipitis* stammt, welche in der Lage ist neben NADPH auch NADH als Cofaktor zu nutzen (Metzger und Hollenberg, 1995), kommt es unter strikt anaeroben Bedingungen zum Erliegen der Xylosefermentation.

Eine Lösung des Problems bestand darin, einen redoxneutralen Stoffwechselweg in *S*. *cerevisiae* einzuführen. In Prokaryonten erfolgt die Umsetzung von Xylose zu Xylulose durch das Enzym Xylose-Isomerase (XI). Für eine vollständige Umsetzung der D-Xylose müsste in *S. cerevisiae* zusätzlich nur das Gen *XI* exprimiert werden, da eine endogene Xylulokinase vorhanden ist. Obwohl eine Xylose-Isomerase in manchen Pilzen nachgewiesen werden konnte (Tomoyeda und Horitsu, 1964; Vongsuvanglert und Tani, 1988; Banerjee *et al.,* 1994; Rawat *et al.,* 1996), war in Eukaryonten nur der Xylose-Abbau über die Enzyme Xylose-Reduktase und Xylitol-Dehydrogenase gezeigt worden. Viele Bemühungen, eine Xylose-Isomerase aus unterschiedlichen Organismen in Hefe heterolog zu exprimieren, schlugen fehl (Gardonyi und Hahn-Hägerdal, 1993). Meistens waren die Enzyme in Hefe nicht funktionell oder sie wurden nicht zu Proteinen synthetisiert (Sarthy *et al.,* 1987; Amore *et al.,* 1989; Moes *et al.,* 1996). Mit hoher Aktivität konnte nur die Xylose-Isomerase aus dem obligat anaeroben Pilz *Piromyces* sp. E2 (Kyper *et al.,* 2003) in Hefe exprimiert werden. Bei heterologer Überexpression dieser eukaryotischen Xylose-Isomerase (Harhangi *et al*., *2003*) war *S*. *cerevisiae* in der Lage, auf Xylose zu wachsen und es auch unter anaeroben Bedingungen zu metabolisieren (Kuyper *et al.,* 2003). Weitere Tests zeigten allerdings, dass das Enzym durch Xylitol, ein Produkt der Xylose-Umsetzung, stark gehemmt wird. Xylitol wird in Hefe unspezifisch aus Xylose mittels Aldose-Reduktasen gebildet.

Die Dokumente ("WO 03/062430" (D1); "Antonius J A Van Maris et al., Kluwer Academic Publishers, DO, Bd. 90, Nr. 4, 11, Oktober 2006, Seiten 391-418" (D2), "Kuyper M et al., Fems Yeast Research, Elsevier Science, Tokyo, NL, Bd. 4, Nr. 6, 1. März 2004, Seiten 655-664" (D4); "Walfridsson M. et al., Applied and Environmental Microbiology, American Society For Microbiology, US, Bd. 62, Nr. 12, 1, Dezember 1996, Seiten 4648-4651" (D5); "Wiedemann Beate et al., Applied and Environmental Microbiology, American Society for Microbiology, US, Bd. 74, Nr. 7, 1. April 2008, Seiten 2043-2050" (D7)) beschreiben alle die Transformation von Hefezellen mit DNA welche das Enzym Xylose-Isomerase aus verschiedenen Organismen (D1 and D4 : aus Piromyces sp, D5 aus Thermus thermophilus, D2 ist ein Review-Artikel, der den Einsatz des Enzyms aus unterschiedlichen Organismen beschreibt) kodiert und die Verwendung dieser Transformanten für die Produktion von z.B. Bioethanol.

US 6,475,768 beschreibt die Verwendung einer prokaryotischen thermophilen Xylose-Isomerase aus *Thermus thermophilus* bzw. Varianten von dieser für die Metabolisierung von Xylose durch Hefen. Das Temperaturoptimum dieses Enzyms bzw. der Varianten liegt bei einer Temperatur (>70°C), die deutlich über der Temperatur liegt, bei der Hefe wächst und metabolisiert (28-35°C), bei Temperaturen über 40°C ist Hefe jedoch inaktiv bzw. stirbt ab. Bei Temperaturen um 30°C ist die Xylose-Isomerase aus *Thermus thermophilus* bzw. auch die Variantenjedoch nahezu inaktiv. Daher erlaubt auch dieses Enzym bzw. dessen Varianten der Hefe keine effektive Xylose-Metabolisierung.

Es besteht also im Stand der Technik Bedarf für Pentose-Isomerasen, insbesondere Xylose-Isomerasen, die eine verbesserte und effizientere Pentose-Umsetzung, insbesondere Xylose-Umsetzung ermöglichen.

Aufgabe der vorliegenden Erfindung ist es daher, verbesserte Pentose-Isomerasen, insbesondere Xylose-Isomerasen, für die Verwendung bei der Xylose-Umsetzung zur Verfugung zu stellen, die insbesondere für Industriehefestämme verwendet werden können.

### Xylose-Isomerase (Xl)-Konstrukte und deren Verwendung

Die Aufgabe wird erfindungsgemäß gelöst durch zur Verfürgung stellen eines Nukleinsäuremoleküls, das eine Nukleinsäuresequenz umfasst, die für eine prokaryotische Xylose-Isomerase (XI) kodiert, für
- die Transformation einer Zelle, bevorzugt für die rekombinante Expression und Herstellung der Xylose-Isomerase,
- die Umsetzung von Xylose zu Xylulose durch die Zelle und/oder
- die Bildung von Folgeprodukten aus Xylose durch die Zelle.

Insbesondere für folgende Verwendungen:
- die Transformation einer Zelle, bevorzugt für die rekombinante Expression/Herstellung der prokaryotischen Xylose-Isomerase,
- die Umsetzung/Verstoffwechselung, dabei insbesondere Fermentation, von Xylose-haltigem Biomaterial,
- die Herstellung von biobasierten Chemikalien,
- die Herstellung von Biobutanol,
- die Herstellung von Bioethanol.

Unter "Folgeprodukten" sollen die Verbindungen verstanden werden, die die Zelle weiter aus der zu Xylulose umgesetzten Xylose produziert, wie beispielsweise biobasierte Chemikalien und Bioalkohole.

Unter "biobasierten Chemikalien" sollen chemische Verbindungen und Stoffe verstanden werden, die aus biologischen Materialien und Rohstoffen (Biomasse) insbesondere unter Verwendung von Mikroorganismen erhalten werden.

Bei den biobasierten Chemikalien kann es sich um eine Verbindung handeln, die ausgewählt ist aus, aber nicht beschränkt ist auf: Milchsäure, Essigsäure, Bernsteinsäure, Äpfelsäure, 1-Butanol, Isobutanol, 2-Butanol, andere Alkohole, Aminosäuren, 1,3-Propandiol, Ethylen, Glycerol, ein β-Lactam-Antibiotikum oder ein Cephalosporin, Alkanen, Terpenen, Isoprenoiden oder dem Vorläufermolekül Amorphadien des Antimalaria-Wirkstoffes Artemisinin.

Die Begriffe "Umsetzung" und "Verstoffwechselung" werden synonym verwendet und bedeuten die Metabolisierung einer Substanz bzw. Umsetzung einer Substanz im Stoffwechsel, hier: die Umsetzung von Xylose, insbesondere die Umsetzung von Xylose zu Xylulose durch eine Zelle, die mit einer erfindungsgemäßen Nukleinsäure transformiert wurde. Eine bevorzugte Umsetzung/Verstoffwechselung ist die Fermentation.

Bei den Nukleinsäuremolekülen handelt es sich um rekombinante Nukleinsäuremoleküle. Des Weiteren umfassen erfindungsgemäße Nukleinsäuremoleküle dsDNA, ssDNA, PNA, CNA, RNA oder mRNA oder Kombinationen davon.

Gemäß einer Ausführungsform betrifft die vorliegende Erfindung die Verwendung eines Nukleinsaüremoleküls nach einem der Ansprüche 1 bis 5.

Die erfindungsgemäße prokaryotische Xylose-Isomerase (XI) stammt von *Clostridium phytofermentans.*

In dieser Erfindung ist es mit einem Testsystem gelungen, eine hoch funktionelle prokaryotische Xylose-Isomerase aus *Clostridium phytofermentans* in der Hefe S. *cerevisiae* zu exprimieren. Es konnte gezeigt werden, dass die gefundene Xylose-Isomerase rekombinanten Hefen eine effiziente Xylose-Verstoffwechselung ermöglicht.

Die erfindungsgemäße prokaryotische Xylose-Isomerase (XI) kann in Zellen, insbesondere eukaryotischen Zellen in einer aktiven Form exprimiert werden. Außerdem ist die erfindungsgemäße prokaryotische Xylose-Isomerase (XI) weniger sensitiv gegenüber einer Inhibierung durch Xylitol als die im Stand der Technik bekannte eukaryotische Xylose-Isomerase aus einem anaeroben Fungus.

Wenn die erfindungsgemäße Nukleinsäuresequenz, die für die prokaryotische Xylose-Isomerase (XI) kodiert, in einer Zelle exprimiert wird, dann wird der Zelle die Fähigkeit verliehen, Xylose in Xylulose umzuwandeln, welche dann weiter metabolisiert werden kann. Dadurch ist die Zelle in der Lage, auf Xylose als Kohlenstoffquelle zu wachsen.

Bevorzugt umfasst die erfindungsgemäße prokaryotische Xylose-Isomerase (XI) eine Aminosäuresequenz, die zu mindestens 95% identisch und noch weiter bevorzugt 99% identisch oder identisch zu der Aminosäuresequenz nach SEQ ID NO. 1 ist.

Die Nukleinsäuresequenz, die für eine prokaryotische Xylose-Isomerase (XI) kodiert, umfasst bevorzugt eine Nukleinsäuresequenz, die zu mindestens 95% identisch und noch weiter bevorzugt 99% identisch oder identisch zu der Nukleinsäuresequenz nach SEQ ID NO. 2 ist.

Die erfindungsgemäßen Nukleinsäuremoleküle umfassen bevorzugt Nukleinsäuresequenzen, die mit der natürlich vorkommenden Nukleinsäuresequenz identisch sind oder für eine Verwendung in einer Wirtszelle Codon-optimiert sind.

Jede Aminosäure wird auf Genebene durch ein Codon verschlüsselt. Jedoch gibt es mehrere verschiedene Codons, die für eine einzelne Aminosäure codieren. Der genetische Code ist folglich degeneriert. Die bevorzugte Codonwahl für eine entsprechende Aminosäure ist von Organismus zu Organismus verschieden. So kann es bei heterolog exprimierten Genen zu Problemen kommen, wenn der Wirtsorganismus bzw. die Wirtszelle einen sehr unterschiedlichen Codon-Gebrauch aufweist. Das Gen kann überhaupt nicht oder nur langsam exprimiert werden. Sogar in Genen von verschiedenen Stoffwechselwegen innerhalb eines Organismus ist ein unterschiedlicher Codon-Gebrauch festzustellen. Von den Glykolyse-Genen aus *S. cerevisiae* ist bekannt, daß sie stark exprimiert werden. Sie weisen einen stark restriktiven Codon-Gebrauch auf. Es ist davon auszugehen, dass durch Anpassung des Codon-Gebrauchs des bakteriellen Xylose-Isomerase-Gens an den Codon-Gebrauch der Glykolyse-Gene aus *S. cerevisiae,* es zu einer Verbesserung des Xylose-Umsatzes in Hefe kommt.

In einer bevorzugten Ausführungsform umfasst die Nukleinsäuresequenz, die für eine prokaryotische Xylose-Isomerase (XI) kodiert, eine Nukleinsäuresequenz, die für die Verwendung in einer Wirtszelle Codon-optimiert ist.

Die Codon-Optimierung besteht im Wesentlichen bevorzugt in einer Anpassung des Codon-Gebrauchs an den Codon-Gebrauch des Wirtsorganismus/Wirtszelle, wie Hefe. Bevorzugt wird der Codon-Gebrauch des bakteriellen Xylose-Isomerase-Gens an den Codon-Gebrauch der Glykolyse-Gene aus *S. cerevisiae* angepasst. Für weitere Details, siehe auch Beispiel 2 und Tabelle 1.

Die Nukleinsäuresequenz, die für eine prokaryotische Xylose-Isomerase (XI) kodiert, umfasst bevorzugt eine Nukleinsäuresequenz, die zu mindestens 95% identisch und noch weiter bevorzugt 99% identisch oder identisch zu der Nukleinsäuresequenz nach SEQ ID NO. 3 ist. Bevorzugt ist das erfindungsgemäß verwendete Nukleinsäuremolekül ein Nukleinsäure-Expressionskonstrukt.

Nukleinsäure-Expressionskonstrukte gemäß der Erfindung sind beispielsweise Expressionskassetten, umfassend ein erfindungsgemäßes Nukleinsäuremolekül, oder Expressionsvektoren, die ein erfindungsgemäßes Nukleinsäuremolekül oder eine Expressionskassette umfassen.

Bevorzugt umfasst ein Nukleinsäure-Expressionskonstrukt Promotor- und Terminatorsequenzen, wobei der Promotor operativ verbunden ist mit der Nukleinsäuresequenz, die für eine prokaryotische Xylose-Isomerase (XI) kodiert.

Bevorzugte Promotorsequenzen sind ausgewählt aus HXT7, verkürzter HXT7, PFK1, FBA1, PGK1, ADH1 und TDH3.

Bevorzugte Terminatorsequenzen sind ausgewählt aus CYC1, FBA1, PGK1, PFK1, ADH1 und TDH3.

Das Nukleinsäure-Expressionskonstrukt kann weiterhin 5' und/oder 3' Erkennungssequenzen und/oder Selektionsmarker umfassen.

Bevorzugt wird der Selektionsmarker ausgewählt aus einem LEU2-Markergen, einem URA3-Markergen oder einem dominanten Antibiotika-Resistenz-Markergen. Ein bevorzugter dominantes Antibiotika-Resistenz-Markergen wird ausgewählt aus Genen, die Resistenzen gegenüber Geneticin, Hygromycin und Nourseothricin verleihen.

Ein Expressionsvektor kann ausgewählt werden aus der Gruppe pRS303X, p3RS305X, p3RS306X, pRS41H, pRS41K, pRS41N, pRS42H, pRS42K, pRS42N oder p423HXT7-6HIS, p424HXT7-6HIS, p425HXT7-6HIS, p426HXT7-6HIS.

Bevorzugt ist die zu transformierenden Zelle ein eukaryotischer Mikroorganismus, bevorzugt eine Hefezelle oder eine Fadenpilzzelle.

Die Hefezelle ist bevorzugt ein Mitglied eines Genus ausgewählt aus der Gruppe aus *Saccharomyces, Kluyveromyces*, *Candida, Pichia, Schizosaccharomyces*, *Hansenula, Kloeckera*, *Schwanniomyces*, *Arxula* und *Yarrowia.*

Die Hefezelle ist weiter bevorzugt ein Mitglied einer Species ausgewählt aus der Gruppe aus S. *cereoisiae,* S. *bulderi*, S. *barnetti*, S. *exiguus,* S. *uvarum*, S. *diastaticus*, *K. lactis*, *K. marxianus* und K *fragilis.*

Die Fadenpilzzelle ist bevorzugt ein Mitglied eines Genus ausgewählt aus der Gruppe aus *Aspergillus, Trichoderma*, *Humicola*, *Acremonium*, *Fusarium*, und *Penicillium.*

### Xolose-vergärende Zellen

Die Aufgabe wird erfindungsgemäß gelöst durch zur Verfügung stellen Zellen nach einem der Ansprüche 6 bis 9, die mit einem Nukleinsäure-Expressionskonstrukt transformiert sind, das für eine prokaryotische Xylose- Isomerase (XI) kodiert.

Eine erfindungsgemäße Zelle ist bevorzugt eine eukaryotische Zelle.

Eine erfindungsgemäße Zelle, insbesondere eine eukaryotische Zelle, ist mit einem Nukleinsäure-Expressionskonstrukt transformiert, welches umfasst:
(a) eine Nukleinsäuresequenz, die für eine prokaryotische Xylose-Isomerase (XI) kodiert,
(b) einen operativ mit der Nukleinsäuresequenz verbundenen Promoter, der die Expression der prokaryotischen Xylose-Isomerase (XI) in der Zelle ermöglicht.

Dabei verleiht die Expression des Nukleinsäure-Expressionskonstrukts der Zelle die Fähigkeit, Xylose direkt zu Xylulose zu isomerisieren.

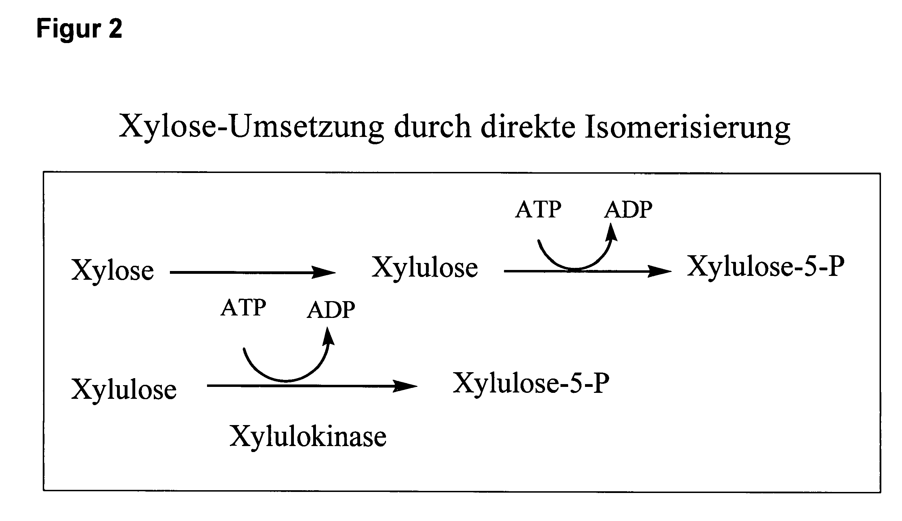

Wie oben diskutiert, kann die erfindungsgemäße prokaryotische Xylose-Isomerase (XI) in Zellen, insbesondere eukaryotischen Zellen, in einer aktiven Form exprimiert werden, so dass die Zellen somit Xylose direkt zu Xylulose isomerisieren können (siehe auch Figur 2).

Außerdem sind die erfindungsgemäßen prokaryotischen Xylose-Isomerase (XI) weniger sensitiv gegenüber einer Inhibierung durch Xylitol als die im Stand der Technik bekannten eukaryotischen Xylose-Isomerasen aus einem anaeroben Fungus.

Die Erfinder haben einen redoxneutralen Stoffwechselweg in *S. cerevisiae* eingeführt, in dem die Umsetzung von Xylose zu Xylulose durch eine Xylose-Isomerase (XI) erfolgt (Fig. 2).

Wenn die erfindungsgemäße Nukleinsäuresequenz, die für die prokaryotische Xylose-Isomerase (XI) kodiert, in einer Zelle exprimiert wird, dann wird der Zelle die Fähigkeit verliehen, Xylose in Xylulose umzuwandeln, welche dann weiter metabolisiert werden kann. Dadurch ist die Zelle in der Lage, auf Xylose als Kohlenstoffquelle zu wachsen.

Die erfindungsgemäße prokaryotische Xylose-Isomerase (XI) stammt bevorzugt aus *Clostridium phytofermentans.* Bevorzugt umfasst die erfindungsgemäße Xylose-Isomerase (XI) eine Aminosäuresequenz, die zu mindestens 70% identisch, bevorzugt mindestens 80% identisch, weiter bevorzugt mindestens 90% identisch, noch weiter bevorzugt mindestens 95% identisch und noch weiter bevorzugt 99% identisch oder identisch zu der Aminosäuresequenz nach SEQ ID NO. 1 ist.

Der Promotor (b) ist bevorzugt ausgewählt aus HXT7, verkürzter HXT7, PFK1, FBA1, PGK1, ADH1 und TDH3.

In einer bevorzugten Ausführungsform ist das Nukleinsäure-Expressionskonstrukt, mit dem eine erfindungsgemäße Zelle transformiert ist, ein erfindungsgemäßes Nukleinsäuremolekül, wie hierin und oben definiert.

Bevorzugt ist die erfindungsgemäße Zelle ein eukaryotischer Mikroorganismus, bevorzugt eine Hefezelle oder eine Fadenpilzzelle.

Eine erfindungsgemäße Hefezelle ist bevorzugt ein Mitglied eines Genus ausgewählt aus der Gruppe aus *Saccharomyces, Kluyveromyces, Candida, Pichia, Schizosaccharomyces, Hansenula, Kloeckera, Schwanniomyces, Arxula* und *Yarrowia.*

Eine erfindungsgemäße Hefezelle ist weiter bevorzugt ein Mitglied einer Species ausgewählt aus der Gruppe aus *S. cerevisiae, S. bulderi, S. barnetti, S. exiguus, S. uvarum, S. diastaticus, K. lactis, K. marxianus* und *K. fragilis.*

Eine erfindungsgemäße Hefezelle ist weiter bevorzugt der Stamm Ethanol Red™ oder Lallemand 1.

Eine erfindungsgemäße Fadenpilzzelle ist bevorzugt ein Mitglied eines Genus ausgewählt aus der Gruppe aus *Aspetgillus, Trichoderma, Humicola, Acremonium*, *Fusarium*, und *Penicillium.*

Bevorzugt ist die erfindungsgemäße Zelle eine m Zellkultur gehaltene Zelle bzw. eine kultivierte Zelle.

Die erfindungsgemäßen Zellen sind mit dem Nukleinsäure-Expressionskonstrukt oder dem Nukleinsäuremolekül, wie hierin definiert, transient oder stabil transformiert.

In einer Ausfiihrungsform exprimiert eine erfindungsgemäße Zelle weiterhin eines oder mehrere Enzyme, die der Zelle die Fähigkeit verleihen, ein oder mehrere weitere Verstoffwechsel ungsprodukte zu produzieren.

Ein solches weiteres Verstoffwechselungsprodukt ist dabei bevorzugt ausgewählt aus, aber nicht beschränkt auf, der Gruppe von biobasierten Chemikalien, wie Milchsäure, Essigsäure, Bernsteinsäure, Äpfelsäure, 1-Butanol, Isobutanol, 2-Butanol, andere Alkohole, Aminosäuren, 1,3-Propandiol, Ethylen, Glycerol, ein ß-Lactam-Antibiotikum oder ein Cephalosporin, Alkanen, Terpenen, Isoprenaiden oder dem Vorläufermolekül Amorphadien des Antimalaria-Wirkstoffes Artemisinin.

Die Aufgabe wird erfindungsgemäß gelöst durch die Verwendung der erfindungsgemäßen Zellen nach Anspruch 10 für die Umsetzung!V erstoffwechselung, dabei insbesondere Fermentation, von Xylose- haltigem Biomaterial und/oder die Herstellung von Bioethanol.

Die Aufgabe wird erfindungsgemäß gelöst durch die Verwendung der entsprechenden erfindungsgemäßen Zellen nach Anspruch 11 für die Umsetzung!Verstoffwechselung, dabei insbesondere Fermentation, von Xylose-haltigem Biomaterial und/oder zur Produktion eines Verstoffwechselungsprodukts.

Wobei das Verstoffwechselungsprodukt bevorzugt ausgewählt ist aus der Gruppe von biobasierten Chemikalien (aber nicht auf diese Gruppe von biobasierten Chemikalien beschränkt ist), wie Milchsäure, Essigsäure, Bernsteinsäure, Äpfelsäure, 1-Butanol, Isobutanol, 2-Butanol, andere Alkohole, Aminosäuren, 1,3-Propandiol, Ethylen, Glycerol, ein ß-Lactam-Antibiotikum oder ein Cephalosporin, Alkanen, Terpenen, Isoprenoiden oder dem Vorläufermolekül Amorphadieu des Antimalaria-Wirkstoffes Artemisinin.

Die Aufgabe wird erfindungsgemäß gelöst durch das zur Verfügung stellen eines Verfahrens zur Produktion von Bioethanol nach einem der Ansprüche 12 bis 14.

Das erfindungsgemäße Verfahren umfasst die folgenden Schritte:
(a) Umsetzung eines Mediums, welches eine Xylose-Quelle enthält, mit einer erfindungsgemäßen Zelle, die Xylose zu Ethanol umsetzt,
(b) optional, Erhalten des Bioethanols.

Das Bioethanol wird beispielsweise durch Isolieren erhalten.

Das Medium kann auch eine andere zusätzliche Kohlenstoffquelle, dabei insbesondere Glukose, enthalten.

Bevorzugt erfolgt die Produktion von Bioethanol mit einer Rate von mindestens 0,03 g Ethanol pro g Hefetrockengewicht und Stunde.

Bevorzugt ist die Ethanolausbeute mindestens 0,3 g Ethanol pro g Xylose.

Die Aufgabe wird erfindungsgemäß gelöst durch das zur Verfügung stellen eines Verfahrens zur Produktion eines Verstoffwechselungsprodukts nach Anspruch 15.

Ein solches weiteres Verstoffwechselungsprodukt ist dabei bevorzugt ausgewählt aus, aber nicht beschränkt auf, der Gruppe von biobasierten Chemikalien wie Milchsäure, Essigsäure, Bernsteinsäure, Äpfelsäure, 1-Butanol, Isobutanol, 2-Butanol, andere Alkohole, Aminosäuren, 1,3-Propandiol, Ethylen, Glycerol, ein ß-Lactam-Antibiotikum oder ein Cephalosporin, Alkanen, Terpenen, Isoprenoiden oder dem Vorläufermolekül Amorphadieu des Antimalaria-Wirkstoffes Artemisinin.

Das erfindungsgemäße Verfahren umfasst die folgenden Schritte:
(a) Umsetzung/Verstoffwechselung, insbesondere Fermentation, eines Mediums, welches eine Xylose-Quelle enthält, mit einer entsprechenden erfindungsgemäßen Zelle, die Xylose umsetzt, um das Verstoffwechselungsprodukt zu produzieren,
(b) optional, Erhalten des Verstoffwechselungsprodukts.

Das Verstoffwechselungsprodukt wird beispielsweise durch Isolieren erhalten.

Das Medium kann auch eine andere zusätzliche Kohlenstoffquelle, dabei insbesondere Glukose, enthalten.

Den Erfindern ist es gelungen, einen redoxneutralen Stoffwechselweg in *S. cerevisiae* einzuführen, in dem die Umsetzung von Xylose zu Xylulose durch eine Xylose-Isomerase (XI) erfolgt (Fig. 2).

In dieser Erfindung ist es mit einem Testsystem gelungen, eine hoch funktionelle prokaryontische Xylose-Isomerase aus *Clostridium phytofermentans* in der Hefe *S. cerevisiae* zu exprimieren. Es konnte gezeigt werden, dass die gefundene Xylose-Isomerase rekombinanten Hefen eine effiziente Xylose-Verstoffwechselung ermöglicht.

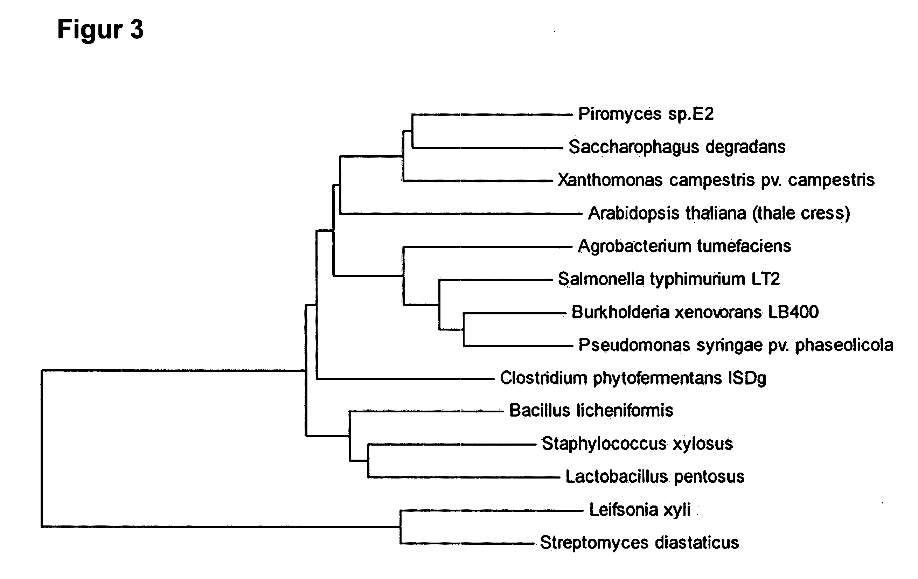

Des Weiteren waren eine Vielzahl von experimentellen Hürden und Schwierigkeiten beim Auffinden einer funktionellen Xylose-Isomerase zu überwinden:
- Für die Konstruktion des Teststammes MKY09 mussten 5 Gene überexprimiert werden.
- Die Auswahl der zu testenden Xylose-Isomerasen war nicht trivial.
- Alle bisher getesteten bakteriellen Xylose-Isomerasen zeigten in Hefe keine bis sehr geringe Aktivität.
- Hoher Aufwand bei der Kultivierung der zu testenden Organismen, die für den Screen benötigt wurden.
- Bei der erfindungsgemäßen Xylose-Isomerase handelt es sich um die erste beschriebene hoch aktive prokaryotische Xylose-Isomerase in Hefe.
- Bei der erfindungsgemäßen Xylose-Isomerase handelt es sich um die erste Xylose-Isomerase des Clusters II (von drei Clustern) von Xylose-Isomerasen (siehe Figure 3), die in Hefen funktionell exprimiert werden konnte.
- Die erfindungsgemäße Xylose-Isomerase wird nur geringfügig durch Xylitol gehemmt.

Über die Schwierigkeiten bezüglich der funktionellen Expression von Xylose-Isomerasen in Hefe gibt es einige Berichte (Gärdonyi and Hahn-Hägerdahl, 2003; sowie darin zitierte Quellen).

Den Erfindern ist es erstmals gelungen, eine prokaryotische Xylose-Isomerase in funktioneller Form in Hefen zu exprimieren, so dass diese dazu befähigt werden, Xylose unter physiologischen Bedingungen und in signifikanten Mengen zu metabolisieren und in Produkte (z.B. Ethanol) umzuwandeln. Wie im Stand der Technik beschrieben, ist dieses nicht trivial. Zahlreiche Versuche dazu wurden unternommen, und alle waren bisher nicht erfolgreich (siehe Sarthy *et al.,* 1987; Amore *et al.,* 1989; Moes *et al.,* 1996, US 6,475,768). Den Erfindern ist es nun gelungen zu demonstrieren, dass gerade die *C. phytofermentans* Xylose-Isomerase, im Gegensatz zu allen anderen bisher bekannten prokaryotischen Enzymen, die Hefe dazu befähigen, Xylose unter physiologischen Bedingungen und in signifikanten Mengen zu metabolisieren und daraus Produkte herzustellen.

Beispiele für lignozellulosische Hydrolysate mit signifikantem Anteil an Xylan (Hayn *et. al.,* 1993):

| | |
|---|---|
| Gras: | 16% |
| Weizenkleie: | 19% |
| Maisabfälle: | 19% |

Die vorliegende Erfindung wird in den folgenden Figuren, Sequenzen und Beispielen weiter verdeutlicht, ohne jedoch darauf beschränkt zu sein, Die zitierten Referenzen sind hiermit durch Bezugnahme vollständig aufgenommen. In den Sequenzen und Figuren zeigt:
SEQ ID NO: 1 die Proteinsequenz des Xylose-Isomerase-ORFs (offener Leserahmen) von C. *phytofermentans,* (siehe auch Genbank Accession Nos. ABX41597 und CP000885 (vom 19. November 2007)),
SEQ ID NO: 2 die Nukleinsäuresequenz des offenen Leserahmens (ORF) der Xylose-Isomerase aus *C. phytofermentans,* (siehe auch Genbank Accession No. CP000885 (vom 19. November 2007)),
SEQ ID NO: 3 die Nukleinsäuresequenz des offenen Leserahmens (ORF) der Xylose-Isomerase aus *C. phytofermentans* in einer Codon-optimierten Form.

**Figur 1****.** *Zusammensetzung der Biomasse*
   Biomasse besteht aus Zellulose, Hemizellulose und Lignin. Die am zweithäufigsten vorkommende Hemizellulose ist ein aus Pentosen, Uronsäuren und Hexosen bestehendes stark verzweigtes Polymer. Die Hemizellulose besteht zu einem großen Anteil aus den Pentosen Xylose und Arabinose.
**Figur 2****.** *Schema für die Umsetzung von D-Xylose in rekombinanter S. cerevisiae über direkte Isomerisierung*
**Figur 3****.** *Stammbaum der verschiedenen Xylose-Isomerasen*
   Gezeigt ist der Stammbaum der getesteten Xylose-Isomerasen. Vergleiche zur Ähnlichkeit der Xylose Isomerasen wurden mit dem Programm "MEGA version 4" durchgeführt.
**Figur 4****.** *Verwendete Vektoren*
   Ausgangsplasmid für die Konstruktion von p426H7-XI-Clos (B) bzw. p426H7-opt.XI-Clos (C) war Plasmid p426HXT7-6HIS (A). Bei dem Vektor p426HXT7-6HIS handelt es sich um ein 2µ Expressionsplasmid, welches einen *URA3*-Marker besitzt. Der offene Leserahmen (ORF) bzw. deren Codon-optimierte Form der erfindungsgemäßen Xylose-Isomerase aus *C*. *phytofermentans* wurde hinter den verkürzten starken *HXT7*-Promotor und dem *CYC1-*Terminator des Plasmids p426HXT7-6HIS kloniert.

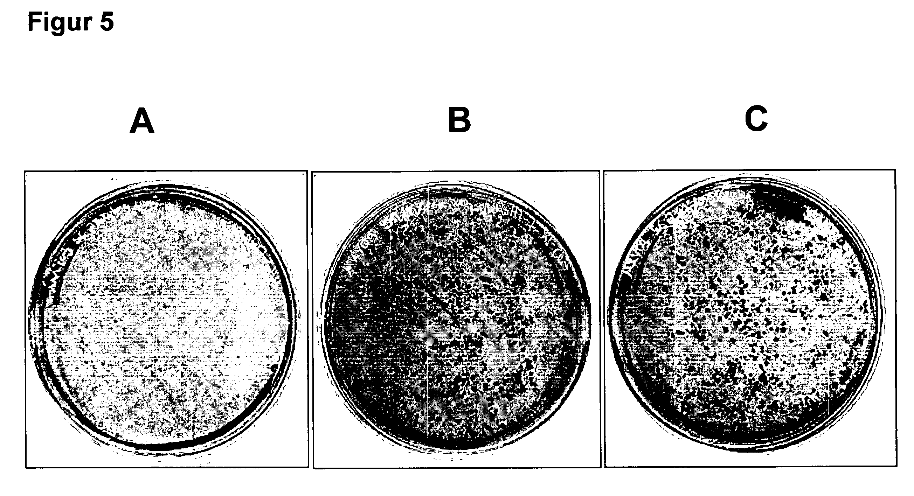

**Figur 5** *Wachstumsverhalten auf Xylose-haltigem Medium unter Verwendung der verschiedenen Xylose-Isomerase-Gene*
   Wachstumstests von rekombinaten *S. cerevisiae* Stämmen, die den bakteriellen D-Xylose-Stoffwechsel mit der Xylose-Isomerase aus *C*. *phytofermentans* enthalten. Wachstumstests wurden auf Agarplatten mit SC-Medium und mit 2 % Xylose als einziger Kohlenstoffquelle durchgeführt. Getestet wurde die native (B) und die Codon-optimierte Form (C) der Xylose-Isomerase aus *C. phytofermentans.* Als Negativ-Kontrolle diente der leere Vektor p426HXT7-6HIS (A).

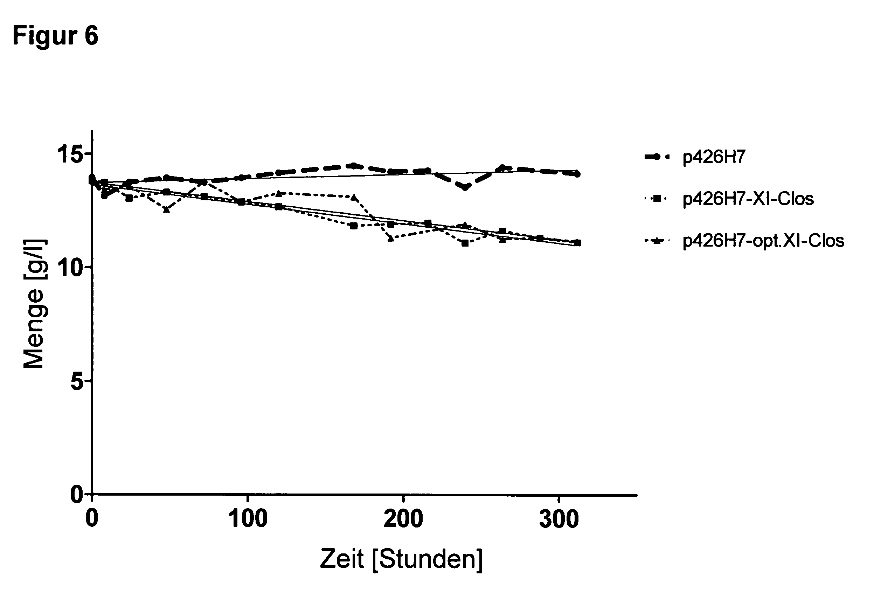

**Figur 6****.** *Xylose-Umsetzung in rekombinanten Hefestämmen unter Verwendung einer bakteriellen Xylose-Isomerase*
   Die Xylose-Umsetzung von rekombinanten Hefezellen MKY09, die die native und die Codon-optimierte Form der Xylose-Isomerase aus *C*. *phytofermentants* enthielten, wurde getestet. Als Vergleich diente der leere Vektor p426HXT7-6HIS. Wachstumskurven wurden in flüssigem SC-Medium mit 1,4% Xylose unter aeroben Bedingungen durchgeführt. HPLC-Proben wurden parallel zur Messung der optischen Dichte bei 600nm entnommen. Siehe auch Tabelle 2, Beispiel 3.

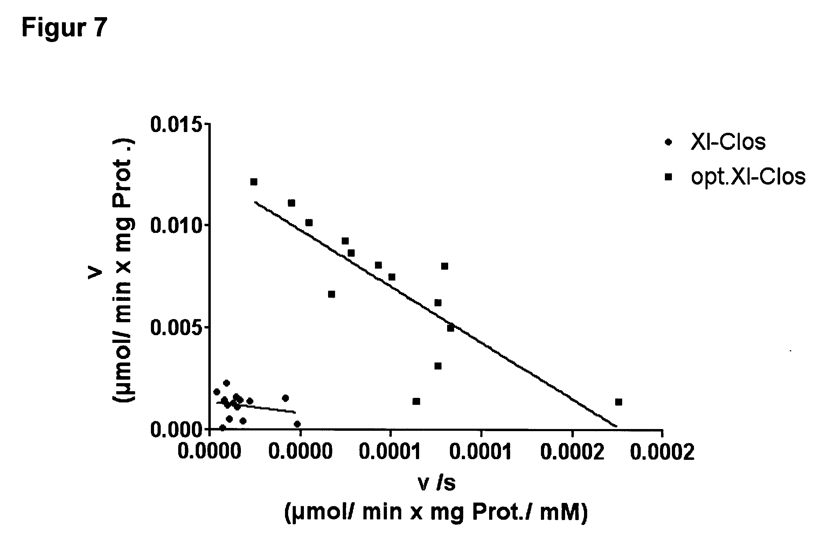

**Figure 7****.** *Enzymkinetiken*
   *Eadie-Hofstee Plot der Xylose Umsetzung der nativen und der Codon-optimierten Xylose-Isomerase aus C. phytofermentans.*
   Der mit dem Plasmid p426H7-XI-Clos bzw. p426H7-opt.XI-Clos transformierte Stamm CEN.PK2-1C wurde über Nacht in synthetischem Komplettmedium mit 2 % Glukose ohne Uracil angezogen. Es wurden Rohextrakte hergestellt und quantitative Enzymtests durchgeführt. Es ist ein repräsentatives Ergebnis gezeigt. Die in Tabelle 3 angegebenen Werte sind Durchschnittswerte aus mindestens 3 unabhängigen Messungen.

### Beispiele

### Methoden

### 1. Stämme und Medien

### - Bakterien

- *E. coli* SURE (Stratagene)
- *E.coli* DH5α (Stratagene)
- *Bacillus licheniformis* (37°C)
- *Agrobacterium tumefaciens* (26°C)
- *Burkholderia xenovorans* (28°C)
- *Clostridium phytofermentans* (30°C, anaerob)
- *Lactobacillus pentosus* (30°C)
- *Leifsonia xyli* (28°C)
- *Pseudomonas syringae pv. phaseolicola* (28°C)
- *Robiginitalea biformata* (30°C)
- *Saccharophagus degradans* (26°C)
- *Salmonella typhimurium LT2* (28°C)
- *Staphylococcus xylosus* (37°C)
- *Streptomyces diastaticus* (28°C)
- *Xanthomonas campestris* (26°C)
andere Organismen
- *Arabidopsis thaliana (genomische DNA)*

### Medien und Anzucht von E. coli

Vollmedium LB:
1% Trypton, 0,5 % Hefeextrakt, 0,5% NaCl, pH 7,5 (siehe Maniatis, 1982).

Für die Selektion auf eine plasmidkodierte Antibiotikaresistenz wurde dem Medium nach dem Autoklavieren 40µg/ml Ampicillin zugesetzt. Feste Nährmedien enthielten zusätzlich 2% Agar. Die Anzucht erfolgte bei 37°C.

### Medien und Anzucht von weiteren Bakterien

Zusammensetzung der Medien und Anzuchtsbedingungen siehe Angaben von DSMZ (Deutsche Sammlung von Mikroorganismen und Zellkulturen, Braunschweig, Deutschland).

### -Hefe

### Stamm CEN.PK2-1C

CEN.PK2-1C (*MATa leu2-3,112 ura3-52 trp1-289 his3-Δ1MAL2-8c SUC2*)

### Stamm MKY09

MKY09 basiert auf dem Stamm CEN.PK2-1 C (*MATa leu2-3,112 ura3-52 trp1-289 his3-Δ1MAL2-8c SUC2, PromTKL1::loxP-Prom-vkHXT7, PromRPE1::loxP-Prom-vkHXT7, PromRKI1::loxP-Prom-vkHXT7, PromGAL2::loxP-Prom-vkHXT7, PromXKS1::loxP-Prom-vkHXT7),* beinhaltet weitere unbekannte Mutationen

### Medien und Anzucht von Hefen

### synthetisches Komplettselektivmedium SC

0,67% Yeast nitrogen base w/o amino acids, pH6,3, Aminosäure/Nukleobase-Lösung, Kohlenstoffquelle in der jeweils angegeben Konzentration

### synthetisches Minimalselektivmedium SM:

0,16% Yeast nitrogen base w/o amino acid and ammonium sulphate, 0,5% Ammoniumsulfat ,20mM Kalium-dihydrogenphosphat, pH6,3, Kohlenstoffquelle in der jeweils angegeben Konzentration

Konzentration der Aminosäuren und Nukleobasen im synthetischen Komplettmedium (nach Zimmermann, 1975): Adenin (0,08mM), Arginin (0,22mM), Histidin (0,25mM), Isoleucin (0,44mM), Leucin (0,44mM), Lysin (0,35mM), Methionin (0,26mM), Phenylalanin (0,29mM), Tryptophan (0,19mM), Threonin (0,48mM), Tyrosin (0,34mM), Uracil (0,44mM), Valin (0,49mM). Als Kohlenstoffquelle wurden L-Arabinose und D-Glucose eingesetzt.

### 2. Plasmide

### Verwendete Plasmide

| Plasmid | Quelle/Referenz | Beschreibung |
|---|---|---|
| p426HXT7-6HIS (=p426H7) | Hamacher *et al.,* 2002 | 2µ-Expressionsplasmid zur Überexpression von Genen und zur Herstellung eines His₆-Epitop; *URA3-*Selektionsmarkergen, verkürzter *HXT7*-Promotor und *CYC1*-Terminator |

Im Rahmen dieser Arbeit konstruierte Plasmide

| Plasmid | Beschreibung |
|---|---|
| p426H7-XI-Agro | Klonierung der *XI* aus *A. tumefaciens* in p426HXT7-6HIS unter Aussparung des His₆-Epitops |
| p426H7-XI-Arab | Klonierung der *XI* aus *A. thaliana* in p426HXT7-6HIS unter Aussparung des His₆-Epitops |
| p426H7-XI-BaLi | Klonierung der *XI* aus *B. licheniformis* in p426HXT7-6HIS unter Aussparung des His₆-Epitops |
| p426H7-XI-Burk | Klonierung der *XI* aus *B. xenovorans* in p426HXT7-6HIS unter Aussparung des His₆-Epitops |
| p426H7-XI-Clos | Klonierung der *XI* aus *C. phytofermentans* in p426HXT7-6HIS unter Aussparung des His₆-Epitops |
| p426H7-opt.XI-Clos | Klonierung der Codon-optimierten *XI* aus *C. phytofermentans* in p426HXT7-6HIS unter Aussparung des His₆-Epitops |
| p426H7-XI-Lacto | Klonierung der *XI* aus *L*. *pentosus* in p426HXT7-6HIS unter Aussparung des His₆-Epitops |
| p426H7-XI- Leif | Klonierung der *XI* aus *L. xyli* in p426HXT7-6HIS unter Aussparung des His₆-Epitops |
| p426H7-opt.XI-Piro | Klonierung der Codon-optimierten *XI* aus Piromyces sp.E2 in p426HXT7-6HIS unter Aussparung des His₆-Epitops |
| p426H7-XI-Pseudo | Klonierung der *XI* aus *P. syringae* in p426HXT7-6HIS unter Aussparung des His₆-Epitops |
| p426H7-XI-Robi | Klonierung der *XI* aus *R. biformata* in p426HXT7-6HIS unter Aussparung des His₆-Epitops |
| p426H7-XI-Saccha | Klonierung der *XI* aus *S. degradans* in p426HXT7-6HIS unter Aussparung des His₆-Epitops |
| p426H7-XI-Salmo | Klonierung der *XI* aus *S. typhimurium* in p426HXT7-6HIS unter Aussparung des His₆-Epitops |
| p426H7-XI-Staph | Klonierung der *XI* aus *S. xylosus* in p426HXT7-6HIS unter Aussparung des His₆-Epitops |
| p426H7-XI-Strep | Klonierung der *XI* aus *S. diastaticus* in p426HXT7-6HIS unter Aussparung des His₆-Epitops |
| p426H7-XI-Xantho | Klonierung der *XI aus X. campestris* in p426HXT7-6HIS unter Aussparung des His₆-Epitops |

### 3. Transformation:

### - Transformation von E. coli

Die Transformation der *E. coli* Zellen erfolgte durch die Elektroporationsmethode nach Dower *et al.* (1988) und Wirth (1993) mittels eines Easyject prima Geräts (EQUIBO).

### - Transformation von S. cerevisiae

Die Transformation von *S*. *cerevisiae* Stämmen mit Plasmid-DNA bzw. DNA-Fragmenten erfolgte nach der Lithiumacetat-Methode von Gietz und Woods (1994).

### 4. Präparation von DNA

### - Isolierung von Plasmid-DNA aus E. coli

Die Isolierung von Plasmid-DNA aus *E. coli* erfolgte nach der Methode der alkalischen Lyse von Birnboim und Doly (1979), modifiziert nach Maniatis *et al.* (1982) oder alternativ mit dem "QIAprep Spin Miniprep Kit" der Firma Qiagen.

Hochreine Plasmid-DNA für Sequenzierungen wurde mit dem "Plasmid Mini Kit" der Firma Qiagen nach Angaben des Herstellers präpariert.

### - Isolierung von Plasmid-DNA aus S. cerevisiae

Die Zellen einer stationären Hefekultur (5ml) wurden durch Zentrifugation geerntet, gewaschen und in 400µl Puffer P1 (Plasmid Mini Kit, Firma Qiagen) resuspendiert. Nach Zugabe von 400µl Puffer P2 und 2/3 Volumen Glasperlen (Ø 0,45mm) erfolgte der Zellaufschluss durch 5-minütiges Schütteln auf einem Vibrax (Vibrax-VXR von Janke & Kunkel oder IKA). Der Überstand wurde mit ½ Volumen Puffer P3 versetzt, gemischt und für 10min auf Eis inkubiert. Nach einer 10-minütigen Zentrifugation bei 13000rpm wurde durch Zugabe von 0,75ml Isopropanol zum Überstand die Plasmid-DNA bei Raumtemperatur gefällt. Die durch Zentrifugation für 30min bei 13000rpm pelletierte DNA wurde mit 70%igem Ethanol gewaschen, getrocknet und in 20µl Wasser resuspendiert. 1µl der DNA wurde für die Transformation in *E. coli* eingesetzt.

### - Kolonie-PCR von B. licheniformis und S. degradans

Geringe Mengen an Zellen wurden mit Hilfe eines Zahnstochers von auf Platte wachsenden Bakterienkulturen entnommen und in ein PCR-Reaktionsgefäß überführt. Nach Zugabe von H₂0, 0,2mM dNTP-Mix, 1xPCR-Puffer (enthält 1,5mM MgCl₂) und je 10pmol der entsprechenden Oligonukleotidprimer erfolgte der Zellaufschluss in einem Thermocycler der Firma Techne bei 99°C für 10 min. Dieser Ansatz wurden direkt als Template in einer PCR-Reaktion eingesetzt. Durch Zugabe von 1U Polymerase wurde die Polymerasekettenreaktion mit einem Gesamtvolumen von 50µl gestartet.

### - Bestimmung der DNA-Konzentration

Die DNA-Konzentration wurde spektralphotometrisch in einem Wellenlängenbereich von 240-300nm gemessen. Liegt die Reinheit der DNA, bestimmt durch den Quotient E₂₆₀ₙₘ/E₂₈₀ₙₘ, bei 1,8, so entspricht die Extinktion E₂₆₀ₙₘ 1,0 einer DNA-Konzentration von 50µg dsDNA/ml (Maniatis *et al.,* 1982).

### - DNA-Amplifikation mittels PCR

### Verwendung des Phusion™ High Fidelity Systems

Die Polymerasekettenreaktion erfolgte in einem Gesamtvolumen von 50µl mit dem "Phusion™ High Fidelity PCR System" der Firma Finnzymes nach Angaben des Herstellers. Jeder Ansatz bestand aus 1-10ng DNA oder 1-2 Hefekolonien als Synthesevorlage, 0,2mM dNTP-Mix, 1xPuffer 2 (enthält 1,5mM MgCl₂), 1U Polymerase und je 100pmol der entsprechenden Oligonukleotidprimer. Die PCR-Reaktion wurde in einem Thermocycler der Firma Techne durchgeführt und die PCR-Bedingungen nach Bedarf wie folgt gewählt:

| | | | |
|---|---|---|---|
| 1. | 1x | 30sec, 98°C | Denaturierung der DNA |
| 2. | 30x | 10sec, 98°C | Denaturierung der DNA |
| | | 30sec, 52-62°C | Annealing/Bindung der Oligonukleotide an die DNA |
| | | 50sec, 72°C | DNA-Synthese/Elongation |
| 3. | 1x | 7min, 72°C | DNA-Synthese/Elongation |

Nach dem ersten Denaturierungsschritt wurde die Polymerase zugegeben ("hot start PCR"). Die Anzahl der Syntheseschritte, die Annealingtemperatur und die Elongationszeit wurden an die spezifischen Schmelztemperaturen der verwendeten Oligonukleotide bzw. an die Größe des zu erwarteten Produkts angepasst. Die PCR-Produkte wurden durch eine Agarosegelelektrophorese überprüft und anschließend aufgereinigt.

### - DNA-Aufreinigung von PCR-Produkten

Die Aufreinigung der PCR-Produkte erfolgte mit dem "QIAquick PCR Purification Kit" der Firma Qiagen nach Herstellerangaben.

### - Gelelektrophoretische Auftrennung von DNA-Fragmenten

Die Auftrennung von DNA-Fragmenten mit einer Größe von 0,15-20kb erfolgte in 0,5-1%igen Agarosegelen mit 0,5µg/ml Ethidiumbromid. Als Gel- und Laufpuffer wurde 1xTAE-Puffer (40mM Tris, 40mM Essigsäure, 2mM EDTA) verwendet (Maniatis *et al.,* 1982). Als Größenstandard diente eine mit den Restriktionsendonukleasen EcoRI und *Hin*dIII geschnittene Lambda-Phagen-DNA. Die DNA-Proben wurden vor dem Auftragen mit 1/10 Volumen Blaumarker (IxTAE-Puffer, 10% Glycerin, 0,004% Bromphenolblau) versetzt und nach der Auftrennung durch Bestrahlung mit UV-Licht (254nm) sichtbar gemacht.

### - Isolierung von DNA-Fragmenten aus Agarosegelen

Das gewünschte DNA-Fragment wurde aus dem TAE-Agarosegel unter langwelligem UV-Licht (366nm) ausgeschnitten und mit dem "QIAquick Gel Extraction Kit" der Firma Qiagen nach Angaben des Herstellers isoliert.

### 5. Enzymatische Modifikation von DNA

### DNA-Restriktion

Sequenzspezifische Spaltung der DNA mit Restriktionsendonukleasen wurden unter den vom Hersteller empfohlenen Inkubationsbedingungen für 1 Stunde mit 2-5U Enzym pro µg DNA durchgeführt.

### 6. Metabolit-Analysen

Zu verschiedenen Zeitpunkten wurden Proben entnommen und bei 4°C für 15 min bei 13000 rpm abzentrifugiert und aus dem Überstand 450 µl entnommen. Die Proteinfällung wurde mit 50%iger Sulfosalizylsäure durchgeführt. 1/10 Volumen Sulfosalizylsäure wurde auf die Proben gegeben, gemischt und für 20 min bei 13000 rpm bei 4°C abzentrifugiert. Der Überstand wurde abgenommen und nach einer weiteren Verdünnung mit Wasser konnten die Proben für die Messung eingesetzt werden. Als Standards dienten Proben mit D-Glukose, D-Xylose, Xylitol, Acetat, Glycerin und Ethanol welche in Konzentrationen von 0,05%w/v, 0,1%w/v, 0,5%w/v, 1,0%w/v und 2,0%w/v eingesetzt wurden.

Die Zuckerkonzentrationen sowie die Ethanolkonzentration wurden mittels BioLC (Dionex) gemessen. Es wurde der Autosampler "AS50", der Säulenofen "TCC-100", der RI-Detektor "RI-101" (Shodex) und die Gradienten-Pumpe "GS50" bei der Messung verwendet.

Die Messung der Proben wurde mit der Säule VA 300/7.7 Nucleogel Sugar 810 H (Macherey-Nagel) durchgeführt. Die Säule wurde bei einer Temperatur von 65°C mit 5 mM H₂SO₄ als Laufmittel und einem Fluss von 0,6 ml min⁻¹ eluiert. Die Auswertung der Daten erfolgte mit dem Programm Chromeleon Version 6.50™ (Version 6.50, Dionex).

### 7. Messung von Enzymaktivitäten in S. cerevisiae

### - Herstellung von Rohextrakten

50 ml Kulturen von Hefezellen wurden bis zur exponentiellen Phase in synthetischem Minimalmedium mit 2% Glucose herangezogen. Die Zellen wurden geerntet, zwei Mal in Tris-HCl Puffer (pH 7,5) gewaschen und mit Hilfe von Glasperlen (Ø = 0,45 mm) für 8 min auf einem Vibrax (Janke & Kunkel, Vibrax-VXR) bei 4°C aufgeschlossen. Zelltrümmer wurden durch 10 min Zentrifugation bei 13000 rpm entfernt. Anschließend wurde der Überstand abgenommen und mit kaltem Tris-HCL Puffer (pH 7,5) auf 2 ml aufgefüllt und als Rohextrakt für die Proteinbestimmung und für die Messung der Enzymaktivitäten bzw. Xylitolinhibierung eingesetzt.

### - Proteinbestimmug

Die Proteinkonzentration wurde mit dem Kit "Roti-Quant" der Firma Carl Roth GmbH + Co. nach Angaben des Herstellers in Anlehnung an Bradford (1976) bestimmt. Als Standard diente dabei Rinderserumalbumin (BSA) in Konzentrationen von 0-100 µg/ml. Nach einer Inkubationszeit von mindestens 5 min bei Raumtemperatur wurden die Proben in Mikrotiterplatten mit einem Mikrotiterplatten Photometer der Firma Moelcular Divces bei OD₅₉₀ vermessen.

### - Messung der Xylose-Isomerase Aktivität

Für die Bestimmung der Xylose-Isomerase Aktivität wurden rekombinante Hefezellen, die den Vektor p426H7-XI-Clos bzw. p426H7-opt.XI-Clos enthielten herangezogen, geerntet und Rohextrakte hergestellt. Als Vergleich dienten rekombinante Hefezellen, die den leeren Vektor p426HXT7-6HIS enthielten. In einem Gesamtvolumen von 1 ml wurde die Umsetzung von 6,25-500 mM Xylose mit 100 µl Rohextrakt, 0,23 mM NADH, 10 mM MgCl₂, 2U Sorbitol-Dehydrogenase in 100 mM Tris-HCl Puffer (pH 7,5) kontinuierlich verfolgt. Die Abnahme von NADH als Messgröße wurde spektralphotometrisch bei einer Wellenlänge von 340 nm bestimmt. Die Reaktion wurde durch Zugabe von Xylose gestartet.

### - Messung der Xylitol-Inhibierung

Für die Bestimmung der Xylitol-Inhibierung der Xylose-Isomerase wurden rekombinante Hefezellen, die den Vektor p426H7-opt.XI-Clos enthielten herangezogen, geerntet und Rohextrakte hergestellt. Als Vergleich dienten rekombinante Hefezellen mit Vektor p426H7-opt.XI-Piro bzw. Vektor p426HXT7-6HIS. In einem Gesamtvolumen von 1 ml wurde die Umsetzung von 6,25-500 mM Xylose mit 100 µl Rohextrakt, 10-100 mM Xylitol, 0,23 mM NADH, 10 mM MgCl₂, 2U Sorbitol-Dehydrogenase in 100 mM Tris-HCl Puffer (pH 7,5) kontinuierlich verfolgt. Die Abnahme von NADH als Messgröße wurde spektralphotometrisch bei einer Wellenlänge von 340 nm bestimmt. Die Reaktion wurde durch Zugabe von Xylose gestartet.

### Beispiel 1: Screen einer (hoch) funktionellen prokaryontischen Xylose-Isomerase

### A) Konstruktion des MKY09

In den Hefestamm CEN.PK2-1C wurden alle Gene des nicht-oxidativen PentosephosphatWeges, sowie die Xylulokinase (*XKS1*) und *GAL2* überexprimiert. Hierzu wurden die endogenen Promotoren gegen den verkürzten *HXT7*-Promotor ausgetauscht. Dieser Stamm wurde MKY09 genannt und für den Screen nach funktionellen Xylose-Isomerasen eingesetzt.

### B) Auswahl der zu testenden Xylose-Isomerasen

Um eine Auswahl für die zu testenden Xylose-Isomerasen zu treffen, wurden Proteinsequenzen von Xylose-Isomerasen aus der Datenbank NCBI BLAST verglichen. Ein Auszug der erhaltenen Xylose-Isomerasen ist in Fig. 3 gezeigt. Es wurden 14 Xylose-Isomerasen aus unterschiedlichen Organismen ausgewählt, um deren Funktionalität in Hefe zu testen.

### C) Durchführung des Screens

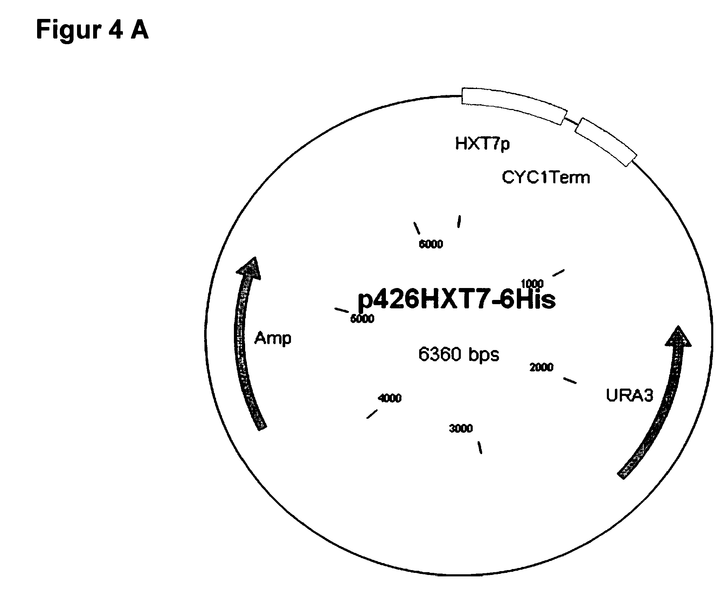

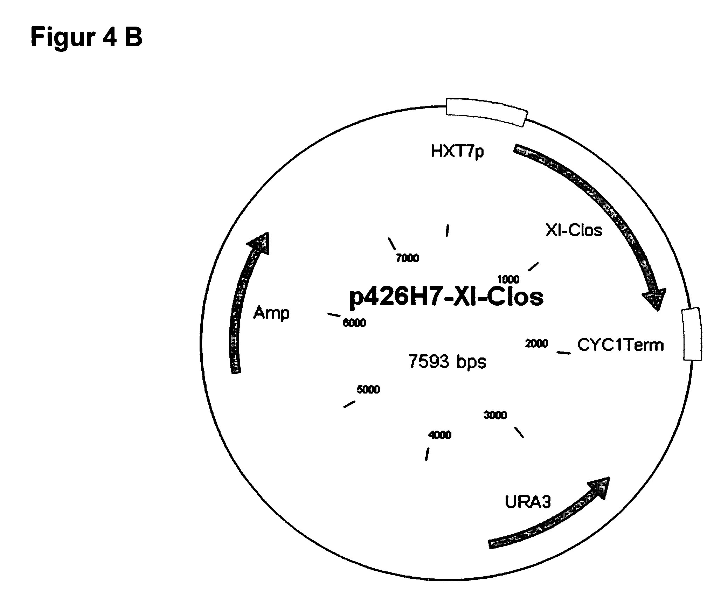

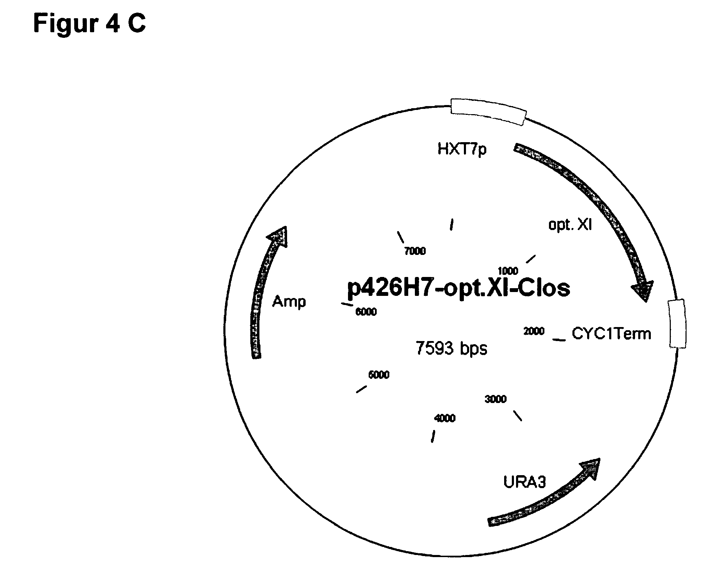

Hierzu wurde genomische DNA aus den Organismen isoliert. Die Zellen wurden herangezogen, geerntet und aufgeschlossen (siehe dazu "Isolierung von Plasmid-DNA aus *S*. *cerevisiae"* bzw. "Kolonie-PCR aus *B. licheniformis* und *S. degradans*). Der offene Leserahmen (ORF) von *XI* aus den genannten Organismen wurde mit Primern amplifiziert, die zusätzlich homologe Bereiche zum *HXT7*-Promotor bzw. *CYC1*-Terminator aufwiesen. Zusammen mit dem *EcoR*I/*BamH*I linearisierten Vektor p426HXT7-6HIS wurden die erhaltenen PCR-Produkte in Hefe transformiert und über *in* vivo-Rekombination in das Plasmid zwischen den *HXT7*-Promotor bzw. *CYC1*-Terminator kloniert (Fig. 4). Die Sequenz der erhaltenen Plasmide wurde über Restriktionsanalyse verifiziert. Weiterhin sollte von den neuen Isomerasen die Funktionalität und deren Effekt auf den Xylose-Umsatz in Hefe untersucht werden. Aus den Organismen *Streptomyces diastaticus* und *Leifsonia xyli* gelang es jedoch nicht, das gewünschte PCR-Produkt mit der Xylose-Isomerase zu amplifizieren. Beide Xylose-Isomerasen konnten folglich nicht auf Funktionalität in Hefe überprüft werden.

### D) Wachstumsverhalten (Platte)

Von den 12 verschiedenen, getesteten Xylose-Isomerasen wurde eine Xylose-Isomerase gefunden, die in Hefestamm MKY09 funktionell war. Rekombinante Hefen, die die Xylose-Isomerase aus *C. phytofermentans* enthielten, zeigten gutes Wachstum auf Xylose-haltigen Platten (Fig.5).

### Beispiel 2: Codon-Optimierung des Genes für den Xylose-Abbau in Hefe

### A) Codon-Optimierung von Genen nach dem Codongebrauch der Glycolysegene aus S. cerevisiae

Der bevorzugte Codongebrauch der Glycolysegene aus *S. cerevisiae* wurde ermittelt und ist in Tabelle 1 aufgelistet. Es wurde der ORF des Genes *XI* aus *C. phytofermentans* Codon-optimiert. Das heisst, die Sequenzen der offenen Leserahmen wurden dem unten angeführten bevorzugten Codon-Gebrauch angepasst. Die Proteinsequenz der Enzyme blieb unverändert. Die Gene wurden bei einer externen Firma synthetisiert und in getrockneter Form in firmeneigenen Hausvektoren geliefert.

Nähere Angaben zu der Synthese von Genen finden sich unter www.geneart.com.

**Tabelle 1: Bevorzugter Codon-Gebrauch der glykolytischen Gene aus S. cerevisiae.**

| Aminosäure | Codon-Gebrauch von Codon-optimierten Genen |
|---|---|
| Ala | GCT |
| Arg | AGA |
| Asn | AAC |
| Asp | GAC |
| Cys | TGT |
| Gln | CAA |
| Glu | GAA |
| Gly | GGT |
| His | CAC |
| Ile | ATT |
| Leu | TTG |
| Lys | AAG |
| Met | ATG |
| Phe | TTC |
| Pro | CCA |
| Ser | TCT |
| Thr | ACC |
| Trp | TGG |
| Tyr | TAC |
| Val | GTT |
| Stopp | TAA |

### B) Einführung des Codon-optimierten Xylose-Isomerase Genes in den Stamm MKY09

Um das Codon-optimierte Xylose-Isomerase Gen in Stamm MKY09 zu testen, musste das Gen in einen Hefevektor subkloniert werden. Hierfür wurde der Codon-optimierte *XI-*ORF mit Primern amplifiziert und in den linearisierten Vektor p426HXT7-6HIS kloniert (siehe "Durchführung des Screens"). Die Sequenz des erhaltenen Plasmids p426H7-opt.XI-Clos wurde über Restriktionsanalyse verifiziert. Zum Test der Funktionalität der Codon-optimierten Isomerase wurde das Plasmid p426H7-opt.XI-Clos in den Stamm MKY09 transformiert. Rekombinante Hefestämme zeigten auf Platten mit Xylose-haltigem Medium gutes Wachstum (Fig.5) Es folgten weitere Charakterisierungen der nativen und der Codon-optimierten *XI* aus *C. phytofermentans.*

### Beispiel 3: Charakterisierung der funktionellen prokaryontischen Xylose-Isomerase

### A) Wachstumsverhalten und Xyloseumsetzung

Das Wachstum des Stammes MKY09 mit der nativen und der Codon-optimierten Xylose-Isomerase aus *C. phytofermentans* wurde in Wachstumstests auf Xylose-haltigem Medium unter aeroben Bedingungen untersucht. Als Vergleich diente der leere Vektor p426HXT7-6HIS.

Die Stämme wurden in SC-Medium mit 0,1% Glukose und 1,4% Xylose herangezogen und mit einer OD₆₀₀ₙₘ = 0,2 in 50ml SC-Medium mit 0,1 % Glukose und 1,4 % Xylose angeimpft. Die Inkubation erfolgte in Schüttelkolben unter aeroben Bedingungen bei 30°C. Es wurden mehrfach Proben zur Bestimmung der optischen Dichte sowie zur Bestimmung der Metabolitzusammensetzung entnommen.

Die Wachstumskurven zeigten, dass die rekombinanten Hefen alle auf Glukose bis zu einer OD600 von 2,5 anwuchsen (Tabelle 2). Nach weiteren 50 h fing der Hefestamm, der die native Xylose-Isomerase aus *C*. *phytofermentans* enthielt, weiter auf Xylose an zu wachsen und erreichte eine End-OD600 von 3,5 mit einer maximalen Wachstumsrate von 0,0058 h⁻¹ auf Xylose-haltigem Medium. Der Hefestamm mit der Codon-optimierten Xylose-Isomerase erreichte ebenfalls eine End-OD600 von 3,5. Die maximale Wachstumsrate betrug 0,0072 h⁻¹. Hefetransformanden mit dem Leer-Vektor p426HXT7-6HIS wiesen kein Wachstum auf Xylose auf und begannen bereits nach 150 h abzusterben.

Die rekombinanten Hefen, die die native Xylose-Isomerase aus *C*. *phytofermentans* bzw. die Codon-optimierte Xylose Isomerase enthielten, setzten in 312 Stunden über 2,6 g Xylose um (Fig. 6).

**Tabelle 2. Bestimmung der maximalen Wachstumsrate auf Xylose (µ)**

| MKY09 transformiert mit Plasmid | max. Wachstumsrate |
|---|---|
| p426H7-XI-Clos | 0,0058 |
| p426H7-opt.XI-Clos | 0,0072 |

Mit dem Versuch konnte gezeigt werden, dass die Einführung der nativen sowie der Codon-optimierten Xylose-Isomerase aus *C. phytofermentans* den rekombinanten *S. cerervisiae* Stämmen das Wachstum auf D-Xylose sowie deren Umsetzung ermöglicht. Durch die Codon-Optimierung der Xylose-Isomerase konnte eine höhere max. Wachstumsrate erreicht werden.

### B) Messung der Xylose-Isomerase Aktivität

Enzymtests wurden direkt nach der Rohextraktherstellung durchgeführt. XI-Aktivität wurde bei 30°C in einem Reaktionsmix (100mM TrisHCl, pH 7,5; 10mM MgCl₂, NADH 0,23 mM; Sorbitiol-Dehydrogenase 2U) mit unterschiedlichen Konzentrationen Rohextrakt durchgeführt. Die Reaktion wurde mit 6,25-500 mM Xylose gestartet.

Die Bestimmung der Enzymkinetik der nativen Form der Xylose-Isomerase ergab einen Kₘ Wert von 61,85 ± 3,41mM und für die Codon-optimierte Form einen Kₘ-Wert von 66,01 ± 1mM (Fig. 7 und Tabelle 3). Wie erwartet, sind somit die Kₘ-Werte gleich, da sie sich nicht signifikant von einander unterscheiden.

Vₘₐₓ(µmol min⁻¹ mg Protein⁻¹) betrug 0,0076 für die native Form der Xylose-Isomerase und 0,0344 für die Codon-optimierte Form (Fig. 7). Folglich konnte durch die Codon-Optimierung des Enzyms Vₘₐₓum über 450% gesteigert werden.

**Tabelle 3**

| CEN.PK2-1C transformiert mit Plasmid | Vmax (µmol min⁻¹ mg Protein⁻¹) | Km (mM) |
|---|---|---|
| p426H7-XI-Clos | 0,0076 | 61,85 ± 3,4 |
| p426H7-opt.XI-Clos | 0,0344 | 66,01 ± 1 |

Der mit dem Plasmid p426H7-XI-Clos bzw. p426H7-opt.XI-Clos transformierte Stamm CEN.PK2-1C wurde über Nacht in synthetischem Komplettmedium mit 2 % Glukose ohne Uracil angezogen. Es wurden Rohextrakte hergestellt und quantitative Enzymtests durchgeführt.

### C) Messung der Xylitol-Inhibierung

Die Bestimmung der Xylitol-Inhibierung der Xylose-Isomerasen erfolgte direkt nach der Rohextraktherstellung. XI-Aktivität wurde bei 30°C in einem Reaktionsmix (100mM TrisHCl, pH 7,5; 10 mM MgCl₂; NADH 0,23 mM; Sorbitiol-Dehydrogenase 2U) mit unterschiedlichen Konzentrationen Rohextrakt durchgeführt. Zusätzlich befanden sich im Reaktionsmix unterschiedliche Konzentrationen an Xylitol (10-100mM). Die Reaktion wurde mit 6,25-500 mM Xylose gestartet.

Kᵢ wurde durch die Gleichung Kₘ' = Kₘ * (1+ i/Kᵢ) ermittelt, wobei i die eingesetzte Xylitol Konzentration und Kₘ' der apparente Kₘ Wert bei entsprechender Xylitol Konzentration darstellen.

Die Bestimmung der Kinetiken der Xylitol-Inhibierung der Xylose-Isomerase aus C. *phytofermentans* ergab einen Kᵢ-Wert von 14,24 ± 1,48mM (Tabelle 4). Wie bereits mehrfach beschrieben (Yamanaka *et al.,* 1969 und darin zitierte Quellen) handelt es sich um eine kompetitive Inhibition.

**Tabelle 4**

| CEN.PK2-1C transformiert mit Plasmid | Ki (mM) |
|---|---|
| p426H7-opt. XI-Piro | 4,67 ± 1,77 |
| p426H7-opt. XI-Clos | 14,51 ± 1,08 |

Der mit dem Plasmid p426H7-opt.XI-Clos bzw. p426H7-opt.XI-Piro transformierte Stamm CEN.PK2-1C wurde über Nacht in synthetischem Komplettmedium mit 2 % Glukose ohne Uracil angezogen. Es wurden Rohextrakte hergestellt und quantitative Enzymtests mit konstanten Xylitol-Konzentrationen von 10-100mM durchgeführt.

Als Vergleich diente die Xylose-Isomerase aus *Piromyces* sp.E2 und der leere Vektor p426HXT7-6HIS. Der ermittelte Kᵢ-Wert der Xylose-Isomerase aus *Piromyces* sp.E2 betrug 4,67 ± 1,77 mM.

Aus den ermittelten Kᵢ-Werten geht hervor, dass die Xylose-Isomerase aus *C*. *phytofermentans* durch Xylitol signifikant geringer inhibiert wird als die Xylose-Isomerase aus *Piromyces* sp.E2.

### D) Beispiele für Vektoren für die Xylose-Isomerase

Ausgangsplasmid für die Konstruktion von p426H7-opt.XI-Clos war das Plasmid p426HXT7-6HIS. Bei dem Vektor handelt es sich um ein 2µ Expressionsplasmid, welches einen *URA3-*Marker besitzt.

Weitere mögliche Expressionsvektoren sind aus der Serie pRS303X, p3RS305X und p3RS306X. Hierbei handelt es sich um integrative Vektoren, die einen dominanten Antibiotika-Marker besitzen. Nähere Angaben zu diesen Vektoren finden sich bei Taxis und Knop (2006).

### Referenzen

Amore, R., Wilhelm, M. und Hollenberg, C. P. (1989) The fermentation of xylose - an analysis of the expression of Bacillus and Actinoplanes xylose isomerase genes in yeast. Appl. Microbiol. Biotechnol. 30:351-357
Banerjee, S., Archana, A. und Satyanarayana, T. (1994) Xylose metabolism in a thermophilic mould Malbranchea pulchella var. sulfurea TMD 8. Curr. Microbiol. 29:349-352
Birnboim, H.C. und J. Doly (1979) A rapid alkaline extraction procedure for screening recombinant plasmid DNA. Nucl. Acids Res. 7: 1513-1523
Dower, W.J., Miller, J.F. und Ragsdale, C.W. (1988) High efficiency transformation of E. coli by high voltage electroporation. Nucl. Acids Res. 16: 6127-6145
Eliasson, A., Christensson, C., Wahlbom, C.F. und Hahn-Hägerdal, B. (2000) Anaerobic xylose fermentation by recombinant Saccharomyces cerevisiae carrying XYL1, XYL2 and XKS1 in mineral medium chemostat cultures. Appl. Environ. Microbiol. 66: 3381-3386
Gárdonyi, M. and Hahn-Hägerdahl, B. (2003) The Streptomyces rubigniosus xylose isomerase is misfolded when expressed in Saccharomyces cerevisiae. Enzym. Microb. Technol. 32, 252-259
Gietz, R.D. und Woods, R.A. (1994) High efficiency transformation in yeast. In: Molecular Genetics of Yeast: Practical Approaches, J.A. Johnston (Ed.). Oxford University Press pp. 121-134
Hahn-Hägerdal, B., Wahlborn, C.F., Gárdonyi, M., van Zyl, W., Otero, R.R.C. und Jönsson, L.J. (2001) Metabolic engineering of Saccharomyces cerevisiae for xylose utilization. Adv. Biochem. Eng. Biotechnol. 73:53-84
Harhangi, H.R. (2003) Xylose metabolism in the anaerobic fungus Piromyces sp. Strain E2 follows the bacterial pathway. Arch Microbiol. 180:134-141.
Hayn, M., Steiner, W., Klinger, R., Steinmuller, H., Sinner, M. and Esterbauer, H. (1993) Basic research and pilot studies on the enzymatic conversion of lignocellulosics. In Bioconversion of forest and agricultural plant residues, ed. Saddler, J. N. (CAB international, Wallingford, UK), pp. 33-72.
Ho, N.W.Y., Chen, Z. und Brainard, A.P. (1998) Genetically engineered Saccharomyces yeast capable of effective cofermentation of glucose and xylose. Appl. Environ. Microbiol.64:1852-1859
Kötter, P. und Ciriacy, M. (1993) Xylose fermentation by Saccharomyces cerevisiae. Appl. Microbiol. Biotechnol. 38:776-783
Kyper, M. Harhangi, H.R., Stave, A.K., Winkler, A.A., Jetten, M.S., de Laat, W.T., den Ridder, J.J.J., Op den Camp, H.J., van Dijken, J.P. und Pronk, J.T. (2003) High-level functional expression of a fungal xylose isomerase: the key to efficient ethanolic fermentation of xylose by Saccharomyces cerevisiae? FEMS Yeast Res. 4:69-78
Maniatis T, Fritsch, E.F und Sambrook, J. (1982) Molecular cloning. A laboratory manual. Cold Spring Harbor Laboratory, New York.
Metzger, M.H. und Hollenberg, C.P. (1994) Isolation and characterization of the Pichia stipitis transketolase gene and expression in a xylose-utilising Saccharomyces cerevisiae transformant. Appl. Microbiol. Biotechnol. 42:319-325
Moes, J.M., Pretorius, I.S. und van Zyl, W.H. (1996) Cloning and expression of the Clostridium thermosulfurogenes D-xylose isomerase gene (xylA) in Saccharomyces cerevisiae Biotechnol. Lett. 18: 269-274.
Rawat, U., Phadthare, S., Deshpande, V. und Rao, M. (1996) A novel xylose isomerase from Neurospora crassa. Biotechnol. Lett. 18:1267-1270
Sarthy AV, McConaughy BL, Lobo Z, Sundstrom JA, Furlong CE, Hall BD, Expression of the Escherichia coli xylose isomerase gene in Saccharomyces cerevisiae. Appl Environ Microbiol. 1987 Sep;53(9):1996-2000
Schaaff-Gerstenschläger, I. und Miosga, T. (1997) The pentose phosphate pathway. In: Yeast Sugar Metabolism: Biochemistry, Genetics, Biotechnology, and Applications. Zimmermann, F.K. und Entian K.-D (Ed.), Kapitel 15. Technomic, Lancaster, PA
Taxis, C. und Knop, M. (2006) System of centromeric, episomal, and integrative vectors based on drug resistance markers for Saccharomyces cerevisiae. BioTechniques 40, No. 1
Tomoyeda, M. und Horitsu, H. (1964) Pentose metabolism in Candida utilis Part I: Xylose isomerase. Agric. Biol. Chem. 28:139-143.
Vongsuvanglert, V. und Tani, Y. (1988) Purification and characterisation of xylose isomerase of a methanol yeast, Candida boidinii, which is involved in sorbitol production from glucose. Agric. Biol. Chem. 52:1818-1824.
Yamanaka, K. (1969) Inhibition of D-xylose isomerase by pentitols and D-lyxose. Arch. Biochem. Biophys. 131, 502-506.
Zimmermann, F. K. (1975) Procedures used in the induction of mitotic recombination and mutation in the yeast Saccharomyces cerevisiae. Mutation Res. 31:71-81.

## Patentansprüche

1. Verwendung eines Nukleinsäuremoleküls, das eine Nukleinsäuresequenz umfasst, die für eine prokaryotische Xylose-Isomerase (XI) kodiert, und die zu mindestens 95% identisch, bevorzugt 99% identisch zu der Nukleinsäuresequenz nach SEQ ID NO. 2 ist, oder die zu mindestens 95% identisch, bevorzugt 99% identisch zu der Nukleinsäuresequenz nach SEQ ID NO. 3 ist, für die Transformation einer Zelle, für die rekombinante Expression und Herstellung der Xylose-Isomerase, und/oder für die Umsetzung von Xylose zu Xylulose durch die Zelle,
wobei die Xylose-Isomerase (XI) aus *Clostridium phytofermentans* stammt und eine Aminosäuresequenz umfasst, die zu mindestens 95% identisch, bevorzugt 99% identisch zu der Aminosäuresequenz nach SEQ ID NO. 1 ist.

2. Verwendung nach Anspruch 1 für
- die Umsetzung und/oder Verstoffwechselung von Xylose-haltigem Biomaterial,
- die Herstellung von biobasierten Chemikalien,
- die Herstellung von Biobutanol und/oder von Bioethanol.

3. Verwendung nach Anspruch 1 oder 2, wobei die Nukleinsäuresequenz SED ID NO. 3, die für eine prokaryotische Xylose-Isomerase (XI) kodiert, eine Nukleinsäuresequenz umfasst, die für die Verwendung in einer Wirtszelle Codon-optimiert ist.

4. Verwendung nach einem der vorhergehenden Ansprüche, wobei das Nukleinsäuremolekül ein Nukleinsäure-Expressionskonstrukt ist, das Promotor- und Terminatorsequenzen umfasst, wobei der Promotor operativ verbunden ist mit der Nukleinsäuresequenz, die für eine Xylose-Isomerase (XI) kodiert, wobei bevorzugt das Nukleinsäure-Expressionskonstrukt weiterhin 5' und/oder 3' Erkennungssequenzen und/oder Selektionsmarker umfasst.

5. Verwendung nach einem der vorhergehenden Ansprüche, wobei die Zelle eine Hefezelle oder eine Fadenpilzzelle ist, wobei bevorzugt die Hefezelle ein Mitglied eines Genus ausgewählt aus der Gruppe aus *Saccharomyces, Kluyveromyces*, *Candida, Pichia, Schizosaccharomyces*, *Hansenula*, *Kloeckera*, *Schwanniomyces*, *Arxula* und *Yarrowia* ist, und bevorzugt ein Mitglied einer Species ausgewählt aus der Gruppe aus *S. cerevisiae, S. bulderi, S. barnetti*, *S. exiguus*, *S. uvarum*, *S. diastaticus*, *K. lactis, K. marxianus* und *K.*
*fragilis* ist, wobei besonders bevorzugt die Fadenpilzzelle ein Mitglied eines Genus ausgewählt aus der Gruppe aus *Aspergillus, Trichoderma, Humicola, Acremonium*, *Fusarium*, und *Penicillium* ist.

6. Zelle, die mit einem Nukleinsäure-Expressionskonstrukt transformiert ist, welches umfasst:
(a) eine Nukleinsäuresequenz, die für eine prokaryotische Xylose-Isomerase (XI) kodiert, wobei die Xylose-Isomerase (XI) aus *Clostridium phytofermentans* stammt und eine Aminosäuresequenz umfasst, die zu mindestens 95% identisch, bevorzugt 99% identisch zu der Aminosäuresequenz nach SEQ ID NO. 1 ist,
(b) einen operativ mit der Nukleinsäuresequenz verbundenen Promoter, der die Expression der Xylose-Isomerase (XI) in der Zelle ermöglicht,
wobei die Expression des Nukleinsäure-Expressionskonstrukts der Zelle die Fähigkeit verleiht, Xylose direkt zu Xylulose zu isomerisieren,
wobei die Zelle eine eukaryotische Zelle ist, und wobei die Zelle mit einem Nukleinsäuremolekül, wie in den Ansprüchen 1 bis 5 definiert, transformiert ist.

7. Zelle nach Anspruch 6, wobei die Zelle eine Hefezelle oder eine Fadenpilzzelle ist, wobei bevorzugt die Hefezelle ein Mitglied eines Genus ausgewählt aus der Gruppe aus *Saccharomyces, Kluyveromyces*, *Candida, Pichia, Schizosaccharomyces, Hansenula, Kloeckera*, *Schwanniomyces*, *Arxula* und *Yarrowia* ist,
und bevorzugt ein Mitglied einer Species ausgewählt aus der Gruppe aus *S. cerevisiae, S. bulderi*, *S. barnetti*, *S. exiguus, S. uvarum*, *S. diastaticus, K. lactis, K. marxianus* und *K*. *fragilis* ist, und wobei bevorzugt die Fadenpilzzelle ein Mitglied eines Genus ausgewählt aus der Gruppe aus *Aspergillus, Trichoderma, Humicola, Acremonium*, *Fusarium*, und *Penicillium* ist.

8. Zelle nach einem der Ansprüche 6 bis 7, welche mit dem Nukleinsäure-Expressionskonstrukt oder dem Nukleinsäuremolekül, wie in den Ansprüchen 1 bis 5 definiert, transient oder stabil transformiert ist.

9. Zelle nach einem der Ansprüche 6 bis 8, welche weiterhin eines oder mehrere Enzyme exprimiert, die der Zelle die Fähigkeit verleihen, weitere Verstoffwechselungsprodukte zu produzieren,
wobei die weiteren Verstoffwechselungsprodukte bevorzugt ausgewählt sind aus der Gruppe der biobasierten Chemikalien, wie Milchsäure, Essigsäure, Bernsteinsäure, Äpfelsäure, 1-Butanol, Isobutanol, 2-Butanol, andere Alkohole, Aminosäuren, 1,3-Propandiol, Ethylen, Glycerol, ein β-Lactam-Antibiotikum oder ein Cephalosporin, Alkanen, Terpenen, Isoprenoiden oder dem Vorläufermolekül Amorphadien des Antimalaria-Wirkstoffes Artemisinin.

10. Verwendung einer Zelle nach einem der Ansprüche 6 bis 8 für die Umsetzung und Verstoffwechselung, insbesondere Fermentation, von Xylose-haltigem Biomaterial und/oder die Herstellung von Bioethanol.

11. Verwendung einer Zelle nach einem der Ansprüche 6 bis 9 für die Umsetzung und Verstoffwechselung, insbesondere Fermentation, von Xylose-haltigem Biomaterial und/oder zur Produktion eines Verstoffwechselungsprodukts,
wobei das Verstoffwechselungsprodukt bevorzugt ausgewählt ist aus der Gruppe der biobasierten Chemikalien, wie Milchsäure, Essigsäure, Bernsteinsäure, Äpfelsäure, 1-Butanol, Isobutanol, 2-Butanol, andere Alkohole, Aminosäuren, 1,3-Propandiol, Ethylen, Glycerol, ein β-Lactam-Antibiotikum oder ein Cephalosporin, Alkanen, Terpenen, Isoprenoiden oder dem Vorläufermolekül Amorphadien des AntimalariaWirkstoffes Artemisinin.

12. Verfahren zur Produktion von Bioethanol, umfassend die Schritte:
(a) Umsetzung eines Mediums, welches eine Xylose-Quelle enthält, mit einer Zelle nach einem der Ansprüche 6 bis 8, die Xylose zu Ethanol umsetzt,
(b) optional, Erhalten des Ethanols, wobei bevorzugt das Medium eine weitere Kohlenstoffquelle, insbesondere Glukose, enthält.

13. Verfahren nach Anspruch 12, wobei die Produktion von Bioethanol mit einer Rate von mindestens 0,03 g Ethanol pro g Hefetrockengewicht und Stunde erfolgt.

14. Verfahren nach einem der Ansprüche 12 bis 13, wobei die Ethanolausbeute mindestens 0,3 g Ethanol pro g Xylose ist.

15. Verfahren zur Produktion eines Verstoffwechselungsprodukts umfassend die Schritte:
(a) Umsetzung, eines Mediums, welches eine Xylose-Quelle enthält, mit einer Zelle nach Anspruch 9, die Xylose umsetzt, um das Verstoffwechselungsprodukt zu produzieren,
(b) optional, Erhalten des Verstoffwechselungsprodukts,
wobei das Verstoffwechselungsprodukt bevorzugt ausgewählt ist aus der Gruppe der biobasierten Chemikalien, wie Milchsäure, Essigsäure, Bernsteinsäure, Äpfelsäure, 1-Butanol, Isobutanol, 2-Butanol, andere Alkohole, Aminosäuren, 1,3-Propandiol, Ethylen, Glycerol, ein β-Lactam-Antibiotikum oder ein Cephalosporin, Alkanen, Terpenen, Isoprenoiden oder dem Vorläufermolekül Amorphadien des AntimalariaWirkstoffes Artemisinin, wobei bevorzugt das Medium eine weitere Kohlenstoffquelle, insbesondere Glukose, enthält.

## Claims

1. Use of a nucleic acid molecule that includes a nucleic acid sequence that codes for a prokaryotic xylose isomerase (XI), and that is at least 95% identical, preferably 99% identical, to the nucleic acid sequence according to SEQ ID NO. 2, or that is at least 95% identical, preferably 99% identical, to the nucleic acid sequence according to SEQ ID NO. 3, for the transformation of a cell, for the recombinant expression and production of xylose isomerase, and/or for the conversion of xylose to xylulose by the cell, wherein the xylose isomerase (XI) comes from *Clostridium phytofermentans* and comprises an amino acid sequence that is at least 95% identical, preferably 99% identical, to the amino acid sequence according to SEQ ID NO. 1.

2. Use according to Claim 1 for
- the conversion and/or metabolism of xylose-containing biomaterial,
- the production of biobased chemicals,
- the production of biobutanol and/or bioethanol.

3. Use according to Claim 1 or 2, wherein the nucleic acid sequence SEQ ID NO. 3 that codes for a prokaryotic xylose isomerase (XI) comprises a nucleic acid sequence that is codon-optimized for use in a host cell.

4. Use according to any one of the preceding claims, wherein the nucleic acid molecule is a nucleic acid expression construct that includes promoter and terminator sequences, wherein the promoter is operatively connected with the nucleic acid sequence that codes for a xylose isomerase (XI), wherein preferably the nucleic acid expression construct also includes 5' and/or 3' recognition sequences and/or selection markers.

5. Use according to any one of the preceding claims, wherein the cell is a yeast cell or a filamentous fungal cell, wherein preferably the yeast cell is a member of a genus selected from the group of *Saccharomyces, Kluyveromyces, Candida, Pichia, Schizosaccharomyces, Hansenula, Kloeckera, Schwanniomyces, Arxula* and *Yarrowia,* and preferably a member of a species selected from the group of *S. cerevisiae, S. bulderi, S. barnetti, S. exiguus, S. uvarum, S. diastaticus, K. lactis, K. marxianus* and *K*. *fragilis,* wherein particularly preferably the filamentous fungal cell is a member of a genus selected from the group of *Aspergillus, Trichoderma, Humicola, Acremonium, Fusarium,* and *Penicillium.*

6. Cell transformed with a nucleic acid expression construct that comprises:
(a) a nucleic acid sequence that codes for a prokaryotic xylose isomerase (XI), wherein the xylose isomerase (XI) comes from *Clostridium phytofermentans* and comprises an amino acid sequence that is at least 95% identical, preferably 99% identical, to the amino acid sequence according to SEQ ID NO. 1,
(b) a promoter that is operatively connected with the nucleic acid sequence that makes possible the expression of the xylose isomerase (XI) in the cell, wherein the expression of the nucleic acid expression construct gives the cell the ability to directly isomerize xylose to xylulose,
wherein the cell is an eukaryotic cell, and wherein the cell is transformed with a nucleic acid molecule as defined in Claims 1 to 5.

7. Cell according to Claim 6, wherein the cell is a yeast cell or a filamentous fungal cell, wherein preferably the yeast cell is a member of a genus selected from the group of *Saccharomyces, Kluyveromyces, Candida, Pichia, Schizosaccharomyces, Hansenula, Kloeckera, Schwanniomyces, Arxula* and *Yarrowia,* and preferably a member of a species selected from the group of *S. cerevisiae, S. bulderi, S. barnetti, S. exiguus, S. uvarum, S. diastaticus, K. lactis, K. marxianus* and *K. fragilis,* and wherein preferably the filamentous fungal cell is a member of a genus selected from the group of *Aspergillus, Trichoderma, Humicola, Acremonium, Fusarium,* and *Penicillium.*

8. Cell according to any one of Claims 6 to 7, which is transiently or stably transformed with the nucleic acid expression construct or the nucleic acid molecule as defined in Claims 1 to 5.

9. Cell according to any one of Claims 6 to 8, which furthermore expresses one or more enzymes that give the cell the ability to produce additional products of metabolism,
wherein the additional products of metabolism are preferably selected from the group of biobased chemicals, such as lactic acid, acetic acid, succinic acid, malic acid, 1-butanol, isobutanol, 2-butanol, other alcohols, amino acids, 1,3-propanediol, ethylene, glycerol, a β-lactam antibiotic or a cephalosporin, alkanes, terpenes, isoprenoids or the precursor molecule amorphadiene of the antimalarial active ingredient artemisinin.

10. Use of a cell according to any one of Claims 6 to 8 for the conversion and metabolism, especially the fermentation, of xylose-containing biomaterial and/or the production of bioethanol.

11. Use of a cell according to any one of Claims 6 to 9 for the conversion and metabolism, especially the fermentation, of xylose-containing biomaterial and/or the production of a product of metabolism,
wherein the product of metabolism is preferably selected from the group of biobased chemicals, such as lactic acid, acetic acid, succinic acid, malic acid, 1-butanol, isobutanol, 2-butanol, other alcohols, amino acids, 1,3-propanediol, ethylene, glycerol, a β-lactam antibiotic or a cephalosporin, alkanes, terpenes, isoprenoids or the precursor molecule amorphadiene of the antimalarial active ingredient artemisinin.

12. Method for production of bioethanol, comprising the following steps:
(a) reacting a medium containing a source of xylose with a cell according to one of Claims 6 to 8, which converts xylose to ethanol,
(b) optionally, obtaining the ethanol, wherein preferably the medium contains an additional carbon source, especially glucose.

13. Method according to Claim 12, wherein the production of bioethanol takes place at a rate of at least 0.03 g ethanol per g yeast dry weight per hour.

14. Method according to any one of Claims 12 to 13, wherein the ethanol yield is at least 0.3 g ethanol per g xylose.

15. Method for production of a product of metabolism comprising the steps of:
(a) reacting a medium containing a source of xylose with a cell according to Claim 9, which converts xylose to produce the product of metabolism,
(b) optionally, obtaining the product of metabolism,
wherein the product of metabolism is preferably selected from the group of biobased chemicals, such as lactic acid, acetic acid, succinic acid, malic acid, 1-butanol, isobutanol, 2-butanol, other alcohols, amino acids, 1,3-propanediol, ethylene, glycerol, a β-lactam antibiotic or a cephalosporin, alkanes, terpenes, isoprenoids or the precursor molecule amorphadiene of the antimalarial active ingredient artemisinin, wherein preferably the medium contains an additional carbon source, especially glucose.

## Revendications

1. Utilisation d'une molécule d'acide nucléique comprenant une séquence d'acide nucléique qui code pour une xylose isomérase (XI) de procaryote et qui est identique à au moins 95 %, de préférence identique à 99 %, à la séquence d'acide nucléique de la SEQ ID NO. : 2, ou qui est identique à au moins 95 %, de préférence identique à 99 %, à la séquence d'acide nucléique de la SEQ ID NO. : 3, pour la transformation d'une cellule destinée à l'expression recombinante et à la fabrication de la xylose-isomérase, et/ou à la conversion de xylose en xylulose par la cellule,
la xylose isomérase (XI) provenant de *Clostridium phytofermentans* et comprenant une séquence d'acides aminés qui est identique à 95 %, de préférence identique à 99 %, à la séquence d'acides aminés de la SEQ ID NO. : 1.

2. Utilisation selon la revendication 1, pour
- la conversion et/ou la métabolisation d'un biomatériau contenant du xylose,
- la fabrication de substances chimiques d'origine biologique,
- la fabrication de biobutanol et/ou de bioéthanol.

3. Utilisation selon la revendication 1 ou 2, dans laquelle la séquence d'acide nucléique de la SEQ ID NO. : 3 codant pour une xylose isomérase (XI) de procaryote comprend une séquence d'acide nucléique, dont les codons sont optimisés pour une utilisation dans une cellule hôte.

4. Utilisation selon l'une quelconque des revendications précédentes, dans laquelle la molécule d'acide nucléique est une construction d'expression d'acide nucléique comprenant une séquence de promoteur et de terminateur, le promoteur étant lié de manière fonctionnelle à la séquence d'acide nucléique qui code pour une xylose isomérase (XI), la construction d'expression d'acide nucléique comprenant en outre de préférence une séquence de reconnaissance d'extrémité 5' et/ou 3' et/ou un marqueur de sélection.

5. Utilisation selon l'une quelconque des revendications précédentes, dans laquelle la cellule est une cellule de levure ou une cellule de champignon filamenteux, la cellule de levure étant de préférence un membre d'un genre choisi dans le groupe comprenant *Saccharomyces, Kluyveromyces*, *Candida, Pichia, Schizosaccharomyces, Hansenula*, *Kloeckera*, *Schwanniomyces*, *Arxula* et *Yarrowia*,
et de préférence un membre d'une espèce choisie dans le groupe comprenant *S. cerevisiae, S. bulderi, S. barnetti*, *S. exiguus, S. uvarum*, *S. diastaticus*, *K. lactis,*
*K. marxianus* et *K.fragilis,* le champignon filamenteux étant, de manière préférée entre toutes, un membre d'un genre choisi dans le groupe comprenant *Aspergillus, Trichoderma*, *Humicola, Acremonium, Fusarium* et *Penicillium.*

6. Cellule, transformée à l'aide d'une construction d'expression d'acide nucléique, laquelle comprend :
(a) une séquence d'acide nucléique codant pour une xylose isomérase (XI) de procaryote, la xylose isomérase (XI) provenant de *Clostridium phytofermentans* et comprenant une séquence d'acides aminés qui est identique à 95 %, de préférence identique à 99 %, à la séquence d'acides aminés de la SEQ ID NO. : 1,
(b) un promoteur lié de manière fonctionnelle à la séquence d'acide nucléique qui permet l'expression de la xylose isomérase (XI) dans la cellule,
l'expression de la construction d'expression d'acide nucléique donnant à la cellule la capacité d'isomériser directement le xylose en xylulose,
la cellule étant une cellule eucaryote, et la cellule étant transformée à l'aide d'une molécule d'acide nucléique telle que définie dans les revendications 1 à 5.

7. Cellule selon la revendication 6, dans laquelle la cellule est une cellule de levure ou une cellule de champignon filamenteux, la cellule de levure étant de préférence un membre d'un genre choisi dans le groupe comprenant *Saccharomyces, Kluyveromyces*, *Candida, Pichia, Schizosaccharomyces, Hansenula*, *Kloeckera*, *Schwanniomyces*, *Arxula* et *Yarrowia,*
et de préférence un membre d'une espèce choisie dans le groupe comprenant *S. cerevisiae, S. bulderi, S. barnetti*, *S. exiguus*, *S. uvarum*, *S. diastaticus*, *K. lactis,*
*K. marxianus* et *K.fragilis,* et dans laquelle la cellule de champignon filamenteux est un membre d'un genre choisi dans le groupe comprenant *Aspergillus*, *Trichoderma*, *Humicola, Acremonium*, *Fusarium,* et *Penicillium.*

8. Cellule selon l'une quelconque des revendications 6 à 7, laquelle est transformée de manière transitoire ou permanente à l'aide de la construction d'expression d'acide nucléique ou de la molécule d'acide nucléique telle que définie dans les revendications 1 à 5.

9. Cellule selon l'une quelconque des revendications 6 à 8, laquelle exprime en outre une ou plusieurs enzymes, qui donnent à la cellule la capacité de produire des produits de métabolisation supplémentaires,
les produits de métabolisation supplémentaires étant de préférence choisis dans le groupe des substances chimiques d'origine biologique telles que l'acide lactique, l'acide acétique, l'acide succinique, l'acide malique, le 1-butanol, l'isobutanol, le 2-butanol, les autres alcools, les acides aminés, le 1,3-propanediol, l'éthylène, le glycérol, un antibiotique β-lactame ou une céphalosporine, les alcanes, les terpènes, les isoprénoïdes ou la molécule amorphadiène qui est le précurseur du principe actif antipaludéen artémisinine.

10. Utilisation d'une cellule selon l'une quelconque des revendications 6 à 8 pour la conversion et la métabolisation, en particulier pour la fermentation, d'un biomatériau contenant du xylose et/ou la fabrication de bioéthanol.

11. Utilisation d'une cellule selon l'une quelconque des revendications 6 à 9 pour la conversion et la métabolisation, en particulier pour la fermentation, d'un biomatériau contenant du xylose et/ou la production d'un produit de métabolisation,
le produit de métabolisation étant de préférence choisi dans le groupe des substances chimiques d'origine biologique telles que l'acide lactique, l'acide acétique, l'acide succinique, l'acide malique, le 1-butanol, l'isobutanol, le 2-butanol, les autres alcools, les acides aminés, le 1,3-propanediol, l'éthylène, le glycérol, un antibiotique β-lactame ou une céphalosporine, les alcanes, les terpènes, les isoprénoïdes ou la molécule amorphadiène qui est le précurseur du principe actif antipaludéen artémisinine.

12. Procédé de production de bioéthanol, comprenant les étapes consistant à :
(a) convertir un substrat contenant une source de xylose à l'aide d'une cellule selon l'une quelconque des revendications 6 à 8 qui convertit le xylose en éthanol,
(b) éventuellement recueillir l'éthanol, le substrat contenant de préférence une source de carbone supplémentaire, en particulier du glucose.

13. Procédé selon la revendication 12, au cours duquel la production de bioéthanol s'effectue à une vitesse d'au moins 0,03 g d'éthanol par gramme de poids sec de levure et par heure.

14. Procédé selon l'une quelconque des revendications 12 à 13, au cours duquel le rendement en éthanol est d'au moins 0,3 g d'éthanol par gramme de xylose.

15. Procédé de production d'un produit de métabolisation comprenant les étapes suivantes :
(a) convertir un substrat contenant une source de xylose à l'aide d'une cellule selon la revendication 9 qui convertit le xylose, afin de produire le produit de métabolisation,
(b) éventuellement recueillir le produit de métabolisation,
le produit de métabolisation étant de préférence choisi dans le groupe des substances chimiques d'origine biologique telles que l'acide lactique, l'acide acétique, l'acide succinique, l'acide malique, le 1-butanol, l'isobutanol le 2-butanol, les autres alcools, les acides aminés, le 1,3-propanediol, l'éthylène, le glycérol, un antibiotique β-lactame ou une céphalosporine, les alcanes, les terpènes, les isoprénoïdes ou la molécule amorphadiène qui est le précurseur du principe actif antipaludéen artémisinine, le substrat contenant de préférence une source de carbone supplémentaire, en particulier du glucose.
